# EUROPEAN PATENT SPECIFICATION

(11) **EP 1 346 229 B1**
(45) Date of publication and mention of the grant of the patent: **16.03.2005**
(21) Application number: 01988064.0
(22) Date of filing: 21.12.2001
(51) Int. Cl.: G01N 33/68

(54) **INVERSE LABELING METHOD FOR THE RAPID IDENTIFICATION OF MARKER/TARGET PROTEINS**
UMGEKEHRTES LABELIERUNGSVERFAHREN ZUR SCHNELLEN IDENTIFIKATION VON MARKER/ZIEL-PROTEINEN
PROCEDE D'ETIQUETAGE INVERSE POUR L'IDENTIFICATION RAPIDE DE PROTEINES CIBLES/DESIDENTIFICATRICES

(30) Priority: 22.12.2000 US 257559 P; 19.11.2001 US 332965 P
(43) Date of publication of application: 24.09.2003
(73) Proprietor: Novartis AG, 4056 Basel (CH); Novartis Pharma GmbH, 1230 Wien (AT)
(72) Inventor: WANG, Yingqi, Karen, East Hanover, NJ 07936 (US); MA, Zhixiang, Langhorne, PA 19047 (US); QUINN, Douglas, Frederick, Morristown, NJ 07960 (US); FU, Emil, W., East Hanover, NJ 07936 (US)
(74) Representative: Grubb, Philip William
(86) International application number: PCT/EP2001/015228
(87) International publication number: WO 2002/052271

(56) References cited:
- WO-A-01/94935
- ROSE, K. ET AL: "A new mass-spectrometric C-terminal sequencing technique finds a similarity between gamma-interferon and alfa2-interferon and identifies a proteolytically clipped gamma-interferon that retains full antiviral activity" THE BIOCHEMICAL JOURNAL, vol. 215, no. 2, 1 November 1983 (1983-11-01), pages 273-277, XP001079683 cited in the application
- ODA, Y. ET AL: "Accurate quantitation of protein expression and site-specific phosphorylation" PROC. NATL. ACAD. SCI., no. 96, 1999, pages 6591-6596, XP001080471 cited in the application
- JI, J. ET AL: "Strategy for qualitative and quantitative analysis in proteomics based on signature peptides" JOURNAL OF CHROMATOGRAPHY, no. 745, 2000, pages 197-210, XP004215299
- CHEN, X. ET AL: "Site-specific mass tagging with stable isotopes in proteins for accurate and efficient protein identification" ANAL. CHEM., vol. 72, no. 6, 15 March 2000 (2000-03-15), pages 1134-1143, XP002207456 cited in the application
- WANG, Y.K. ET AL: "Inverse 18 O labeling mass spectrometry for the rapid identification of marker/target proteins" ANALYTICAL CHEMISTRY, vol. 73, no. 15, 1 August 2001 (2001-08-01), pages 3742-3750, XP001061446 USA

## Description

### 1. Field of the Invention

This invention relates to methods for identifying specific proteins in complex protein mixtures. In particular, the methods of the present invention relate to the rapid identification of differentially expressed proteins from two different samples, e.g., different tissues; different cell types or different cell states, using mass spectrometry.

### 2. Description of the Related Art

It has been well established that most disease processes and disease treatments are manifest at the protein level. The mechanisms of action for most of the pharmaceuticals on the market are indeed mediated through proteins. Comparative analysis of protein profiles from normal and disease states, with or without drug treatment, can facilitate the systematic studies of proteins involved in any biological system or disease, revealing new insights into disease mechanisms, identifying new targets, providing information on drug-action mechanisms and toxicity, and identifying surrogate markers. It is believed that proteomic studies will lead to important new insights into disease mechanisms and improved drug-discovery strategies for the discovery of novel therapeutics.

The most common technology platform for proteomic studies to date is the integrated use of two-dimensional (2D) gel electrophoresis for profiling proteins and mass spectrometry for protein analysis and identification as described, e.g., in Quadroni, et al., *Electrophoresis,* 1999, 20:664-677. Protein mixtures derived from cells or tissues of normal or disease states are separated on 2D PAGE and visualized via staining. Quantitative comparisons of images can be made after the images of the displayed proteins are digitally scanned into a computer. The spots that are either unique or those that are differentially expressed are then identified. Following excision of the spots and *in situ* digestion, a variety of mass spectrometric techniques can be used to obtain peptide fingerprint and peptide sequence information which are used to search a sequence database to identify the proteins. As these proteins are disease specific, each could potentially become a new target for drug discovery or be used as a disease marker. At the present time, 2D-PAGE is still the most comprehensive method for displaying proteins. 2D gels have been shown to be highly reproducible since the introduction of immobilized pH gradient (IPG) strips for the first dimensional separation. It is capable of resolving thousands of proteins and, when stained with silver or fluorescent dyes, it provides a sensitive method for quantitating protein expression. Nonetheless, there are still certain shortcomings with the technique. Chief among them is its inability to display all protein components, such as membrane proteins, proteins with extreme pIs, and proteins of low copy numbers. Inadequate resolving power is another pitfall with the technique. Up to 20-40% of all spots may contain more than one protein, which makes quantitative comparison of protein expressions and interpretation of experiments extremely difficult. Although a lot of progress has been made over the last few years, proteomics using 2D gels is still viewed as a difficult technology in terms of automation and throughput. 2D gel electrophoresis, staining, and image analysis are just some of the steps that remain to be fully automated before the process can be truly called high throughput. Alternatives to this technology, particularly to replace the use of 2D gels, are being explored in the hope of achieving better throughput and higher sensitivity.

One approach that omits 2D gels is the use of multi-dimensional liquid phase separation techniques such as chromatography and/or solution isoelectric focusing to partially resolve mixtures of proteins or their digested peptide products as described, e.g., in Eng et al., *J. Am. Soc. Mass Spectrom.* 1994, 5:976-989; McCormack et al., *Anal*. Chem. 1997, 69:767-776; Opiteck et al., *Anal. Chem.* 1997,69:2283-2291; Opiteck et al., *Anal. Chem.* 1997, 69:1518-1524; Opiteck et al., *Anal. Biochem.* 1998,258:349-361; Kojima et al., *J. Chromatogr.* 1982, 239:565-570; Isobe et al., *J. Chromatogr.* 1991, 588:115-123; Wall et al., *Anal. Chem.* 2000, 72:1099-1111; Jensen et al., *Anal. Chem.* 1999, 71:2076-2084; and Pa 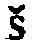 a-Toli 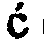 et al., *J. Am. Chem. Soc.* 1999, 121:7949-7950. Mass spectrometry (MS) with additional resolving power, is used to identify the simplified mixture. Since separation occurs in the liquid phase, the automation potential is much higher than the gel-based platform. When running at preparative scale, sample loading is significantly larger than what is achievable with 2D PAGE. In addition, this approach reduces the protein / peptide recovery losses associated with 2D-gel technology since the final separated proteins / peptides are in solution. One negative aspect is that the quantitative information gained from 2D-gel imaging is not yet achievable with these methodologies.

Isotope dilution has long been used for quantitative analysis of drug in biological materials. An internal standard, which is isotopically different in structure, is added to the samples to achieve accurate quantitation of a particular compound. Because of the internal standard, variables such as sample loss during sample preparation, matrix effects, detection interferences, and others, are no longer issues for accurate quantitation. In order to apply the same principle to relative protein quantitation, efforts have been made towards the development of protein tagging or isotope labeling methodologies. Labeling of a pool of proteins can be carried out metabolically or chemically. When evaluating differential expression of proteins, two pools of proteins (e.g., a normal vs. a disease state), one labeled (with heavy isotope) and the other not (i.e., with natural, light isotope), are mixed, proteolyzed and analyzed. Each pair of peptide signals, with and without label, becomes the internal standard for each other and enables the quantitative comparison of protein differential expression. While the peptide fingerprint and peptide sequence information obtained from MS analysis provides the identification of proteins, the label offers a means to differentiate the two populations and perform accurate quantitation on every protein. Protein profiling, quantification, and identification are therefore performed in a single step. Oda et al., *Proc. Natl. Acad. Sci. USA* 1999, 96:6591-6596, have demonstrated such an approach where proteins are metabolically labeled during cell culture in a ¹⁵N-enriched culture media. Similar strategies may also be applied via amino acid specific labeling of proteins achieved metabolically during cell culture cultivation as described, e.g., in Chen et al., *Anal. Chem.* 2000,72:1134-1143. Gygi et al., *Nature Biotech.* 1999, 17:994-999, have developed a chemical derivatization scheme, termed isotope-coded affinity tagging (ICAT) to carry out labeling on all cysteine-containing proteins. With the approach, relative protein quantitation is achieved through the use of two isotopically different, light and heavy tags. The method has been applied successfully in a number of cellular systems to obtain quantitative comparison of protein expression. The built-in affinity tag in the label enables the reduction of peptide mixture complexity by selectively enriching only the cysteine-containing peptides. It however also risks losing information on non-cysteine-containing proteins and information regarding protein post-translational modifications. Data analysis can be tedious with these methods. There is no built-in mechanism to perform subtractive analysis to achieve a quick focus on proteins that change the most in expression. Rather, each peptide pair of light and heavy tags has to be identified and relative quantitation performed for all proteins before a rank order can be obtained. Dynamic range is another limiting factor with the methods. Signals from peptides with both light and heavy isotope tags have to be quantitatively detected in order to obtain accurate quantitation of protein expression. In an extreme situation where only one signal of the pair is detected, the signal can be confused as a chemical background or from a non-cysteine-containing peptide rather than from a protein that has been highly differentially expressed. In addition, the labeling methods mentioned here all require special reagents (custom-made chemicals or isotopically enriched culture media) and extra effort to introduce the labels, which may or may not be readily accessible to a protein analytical lab or an MS lab.

While the above methods permit the identification and quantitation of differentially expressed proteins in complex protein mixtures, these methods are deficient in either speed/throughput, sensitivity, the ability to cover all proteins or the ability to identify extreme changes in expression or protein covalent changes. Accordingly, it would be desirable to provide a method for identifying various classes of differentially expressed proteins in complex protein mixtures that is rapid, high throughput, sensitive and capable to identify all changes in protein expression (quantitative or qualitative) unambiguously.

### SUMMARY OF THE INVENTION

The present invention relates to a novel procedure of performing protein labeling for comparative proteomics termed inverse labeling which is utilized to identify differentially expressed proteins within complex protein mixtures. In particular, the method of the present invention allows the identification of differentially expressed proteins in two different samples, for example, different tissue or cell types, disease or developmental stages.

The method as described herein below, overcomes disadvantages inherent in currently available methods in that it provides rapid, high throughput, sensitive, reliable and unambiguous identification of various classes of differentially expressed proteins.

In one aspect, a method for identifying a differentially expressed protein in two different samples containing a population of proteins is provided which comprises a) providing two equal protein pools from each of a reference sample and an experimental sample; b) labeling the protein pools with a substantially chemically identical isotopically different labeling reagent for proteins, wherein one pool from each of the reference and experimental pools is labeled with an isotopically heavy protein labeling reagent to provide an isotopically heavy-labeled reference pool and an isotopically heavy-labeled experimental pool, and wherein the remaining reference and experimental pools are labeled with an isotopically light protein labeling reagent to provide an isotopically light-labeled reference pool and an isotopically light labeled experimental pool; c) combining the isotopically light-labeled reference pool with the isotopically heavy-labeled experimental pool to provide a first mixture; d) combining the isotopically heavy-labeled reference pool with the isotopically light-labeled experimental pool to provide a second mixture; e) detecting the labeled proteins from each of the two mixtures; and f) comparing the labeling pattern obtained for the labeled proteins in the first and second mixtures, wherein an inverse labeling pattern of a protein in the second mixture compared with the labeling pattern of the protein in the first mixture is indicative of the differentially expressed protein in the two different samples.

In another aspect, a method for identifying a differentially expressed protein in two different samples containing a population of proteins is provided which comprises
a) providing two equal protein pools from each of a reference sample and an experimental sample; b) proteolyzing each protein pool during labeling of each of the protein pools with isotopically-labeled water, wherein one pool from each of the reference and experimental pools is labeled with ¹⁸O-water to provide an ¹⁸O-labeled reference pool and an ¹⁸O-labeled experimental pool, and wherein the remaining reference and experimental pools are labeled with ¹⁶O-water to provide an ¹⁶O-labeled reference pool and an ¹⁶O-labeled experimental pool; c) combining the ¹⁶O-labeled reference pool with the ¹⁸O-labeled experimental pool to provide a first mixture containing ¹⁶O- and ¹⁸O-labeled peptides; d) combining the ¹⁸O-labeled reference pool with the ¹⁶O-labeled experimental pool to provide a second mixture containing ¹⁸O- and ¹⁶O-labeled peptides; e) detecting the labeled peptides from each of the two mixtures; and f) comparing the labeling pattern obtained for the labeled peptides in the first and second mixtures, wherein an inverse labeling pattern obtained for a peptide in the second mixture compared with the labeling pattern obtained for the peptide in the first mixture is indicative of the differentially expressed protein from which the peptide originated.

In another aspect, a method for identifying a differentially expressed protein in two different samples containing a population of proteins is provided which comprises
a) providing two equal protein pools from each of a reference sample and an experimental sample; b) proteolyzing the proteins in each of the protein pools to provide peptide pools; c) labeling each peptide pool with isotopically-labeled water, wherein one peptide pool from each of the reference and experimental pools is labeled with ¹⁸O-water to provide an ¹⁸O-labeled reference peptide pool and an ¹⁸O-labeled experimental peptide pool, and wherein the remaining reference and experimental peptide pools are labeled with ¹⁶O-water to provide an ¹⁶O-labeled reference peptide pool and an ¹⁶O-labeled experimental peptide pool; d) combining the ¹⁶O-labeled reference pool with the ¹⁸O-labeled experimental pool to provide a first mixture containing ¹⁶O- and ¹⁸O-labeled peptides; e) combining the ¹⁸O-labeled reference pool with the ¹⁶O-labeled experimental pool to provide a second mixture containing ¹⁸O- and ¹⁶O-labeled peptides; f) detecting the labeled peptides from each of the two mixtures; and g) comparing the labeling pattern for the labeled peptides in the first and second mixture, wherein an inverse labeling pattern obtained for a peptide in the second mixture compared with the labeling pattern obtained for the peptide in the first mixture is indicative of the differentially expressed protein from which the peptide originated.

In yet another aspect, a method for identifying a differentially expressed protein in two different samples containing a population of proteins is provided which comprises
a) providing two equal protein pools from each of a reference sample and an experimental sample wherein one pool from each of the reference and experimental pools is produced by cultivation in a culture medium containing an isotopically heavy-labeled assimilable source to provide an isotopically heavy-labeled reference pool and an isotopically heavy-labeled experimental pool, and wherein the remaining reference and experimental pools are produced by cultivation in a culture medium containing an isotopically light-labeled assimilable source to provide an isotopically light-labeled reference pool and an isotopically light-labeled experimental pool; b) combining the isotopically light-labeled reference pool with the isotopically heavy-labeled experimental pool to provide a first protein mixture; c) combining the isotopically heavy-labeled reference pool with the isotopically light-labeled experimental pool to provide a second protein mixture; d) detecting the labeled proteins from each of the two mixtures; and e) comparing the labeling pattern obtained for the labeled proteins in the first and second mixture, wherein an inverse labeling pattern of a protein in the second mixture compared with the labeling pattern of the protein in the first mixture is indicative of the differentially expressed protein in the two different samples.

### BRIEF DESCRIPTION OF THE DRAWINGS

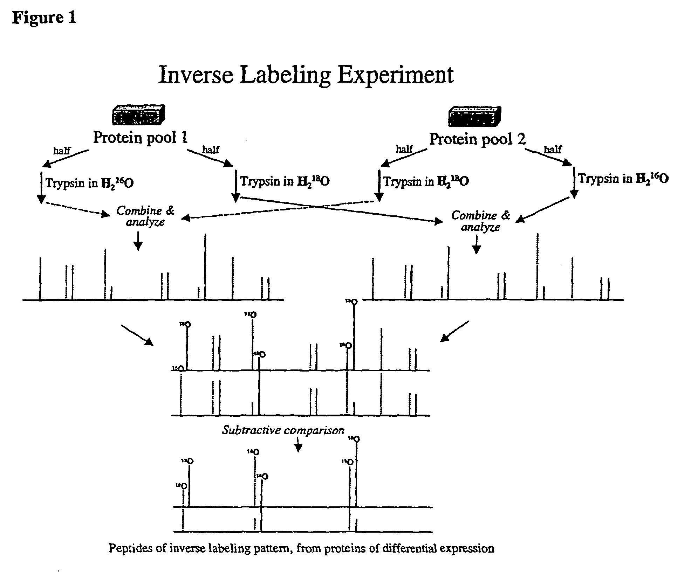

Figure 1. The inverse labeling method for rapid identification of marker/target proteins. For illustration purposes, proteins that remain unchanged in the two protein pools are shown in equal abundance. (In practice, they may not necessarily be present in equal abundance; rather, they may be present at a constant ratio that is not equal to one.) Protein proteolytic ¹⁸O-labeling is used in this schematic diagram for illustration.

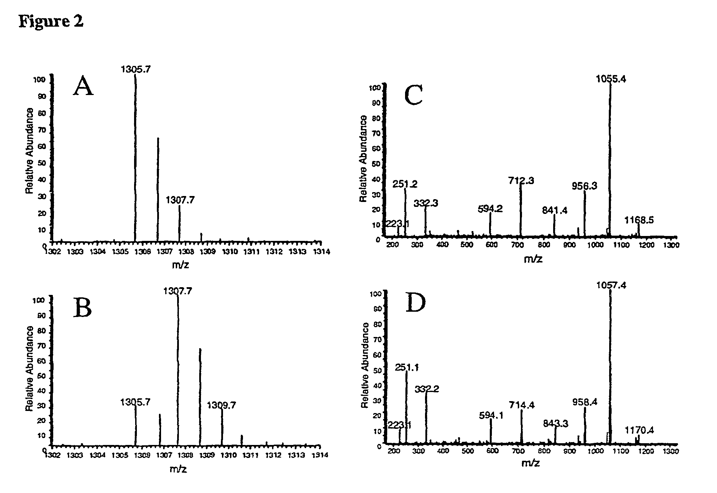

Figure 2. Liquid Chromatography/Mass Spectrometry (LC/MS) detection of an inverse ¹⁸O-labeled BSA tryptic peptide. (A): MS of the ¹⁶O-control- ¹⁸O-"treated" sample; (B): MS of the ¹⁸O-control- ¹⁶O-"treated" sample; (C): MS/MS of the peptide in (A); and (D): MS/MS of the peptide in (B). A 2-Da mass shift between (A) and (B) on the most abundant isotopic ions indicates a significant differential expression of the protein. The mass shift is further verified/confirmed in the MS/MS spectra (C) and (D) by the 2-Da shift of all Y ions, which also helps to identify Y ions and B ions and thus helps in the interpretation of the spectra. The BSA protein is exclusively identified from database searching using the Y ions (those with a 2-Da shift).

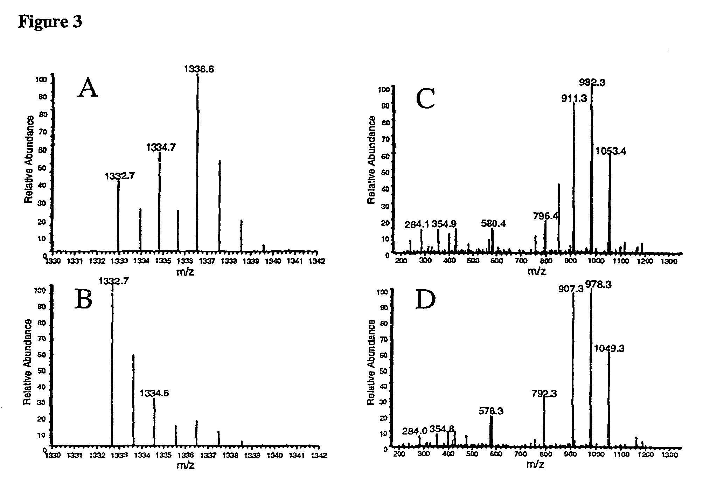

Figure 3. LC/MS detection of an inverse ¹⁸O-labeled aldolase tryptic peptide. (A): MS of the ¹⁶O-control- ¹⁸O-"treated" sample; (B): MS of the ¹⁸O-control- ¹⁶O-"treated" sample; (C): MS/MS of the peptide in (A); and (D): MS/MS of the peptide in (B). A 4-Da mass shift between (A) and (B) on the most abundant isotopic ions indicates a significant differential expression of the protein. The mass shift is further verified/confirmed in the MS/MS spectra (C) and (D) by the 4-Da shift of all Y ions, which also helps to identify Y ions and B ions and thus helps in the interpretation of the spectra. Aldolase protein is exclusively identified from database searching using the Y ions (those with a 4-Da shift).

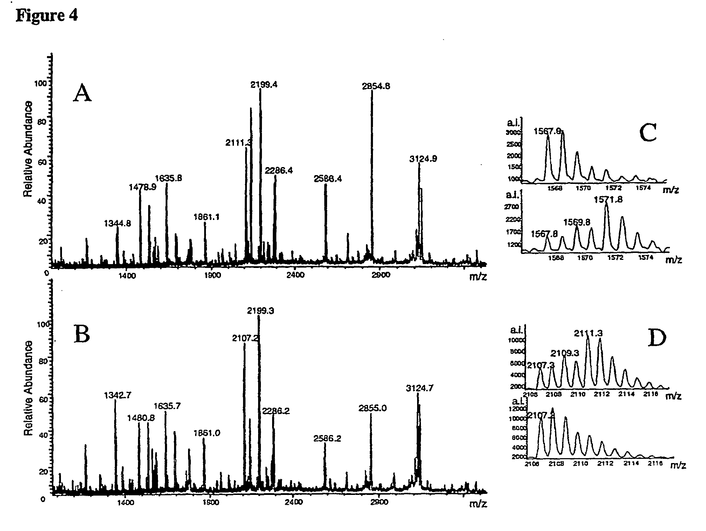

Figure 4. MALDI TOF detection of inverse ¹⁸O-labeled tryptic digests of the 8-protein mixtures. (A): ¹⁶O-control-¹⁸O-"treated" sample; (B): ¹⁸O-control- ¹⁶O-"treated" sample; (C): monoisotopic patterns of a BSA peptide MH⁺ 1567.9 in (A) (upper) and (B) (lower); and (D): monoisotopic patterns of an aldolase peptide MH⁺ 2107.3 in (A) (upper) and (B) (lower). The mass shifts or ¹⁶O-/¹⁸O-intensity ratio reversal indicates differential expression of the proteins: "down-regulation" of BSA and "up-regulation" of aldolase.

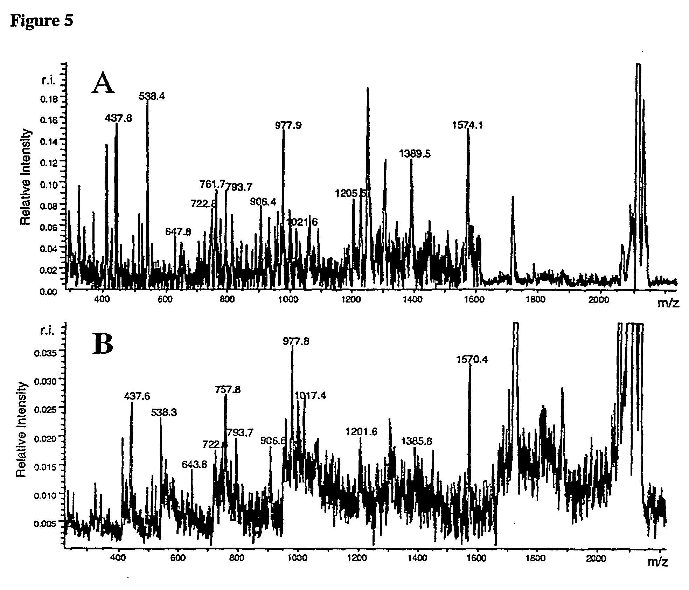

Figure 5. MALDI PSD spectra of an inverse ¹⁸O-labeled aldolase tryptic peptide MH⁺ 2107.3. (A): in the ¹⁶O-control - ¹⁸O-"treated" sample; and (B): in the ¹⁸O-control - ¹⁶O-"treated" sample. The 4-Da mass shift observed on the molecular ion in Figure 4 (D) is further verified/confirmed in the PSD spectra by the 4-Da shift of all Y ions. This also helps to identify Y ions and B ions and thus helps in the interpretation of the PSD spectra. The aldolase protein is exclusively identified from database searching using the Y ions (those with a 4-Da shift).

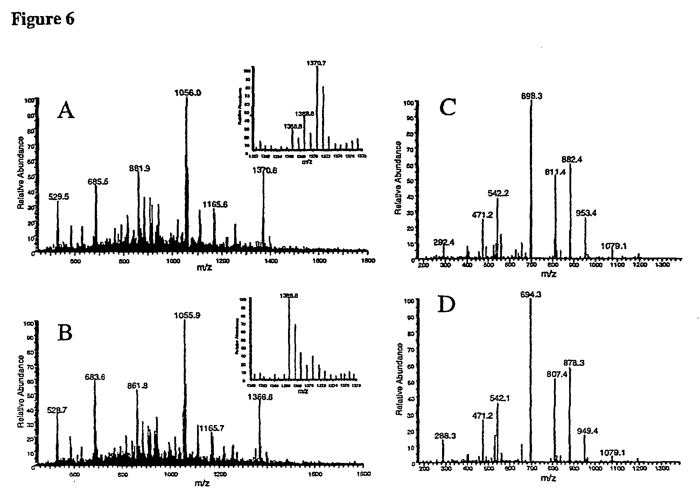

Figure 6. LC/MS detection of a PTP (protein tyrosine phosphatase) tryptic peptide from a CHO cell lysate spiked with PTP-1B. (A): MS of the ¹⁶O-PTP10- ¹⁸O-PTP30 sample; (B): MS of the ¹⁸O-PTP10 - ¹⁶O-PTP30 sample; (C): MS/MS of the peptide in (A) in-set; and (D): MS/MS of the peptide in (B) in-set, where PTP10 is a 0.25 mg CHO cell lysate spiked with 10 pmol of PTP-1B; PTP30 is a 0.25 mg CHO cell lysate spiked with 30 pmol of PTP-1B. After spiking, the protein mixtures are proteolyzed, and subsequently inverse ¹⁸O-labeled to form the two mixtures A and B. A 4-Da mass shift between (A) and (B) (inserts) on the most abundant isotopic ions indicates a significant "differential expression" of the protein. The mass shift is further verified/confirmed in the MSlMS spectra by the 4-Da shift of all Y ions, which also helps to identify Y ions and B ions and thus helps in the interpretation of the MS/MS spectra. PTP-1B protein is exclusively identified from database searching using the Y ions (those with a 4-Da shift).

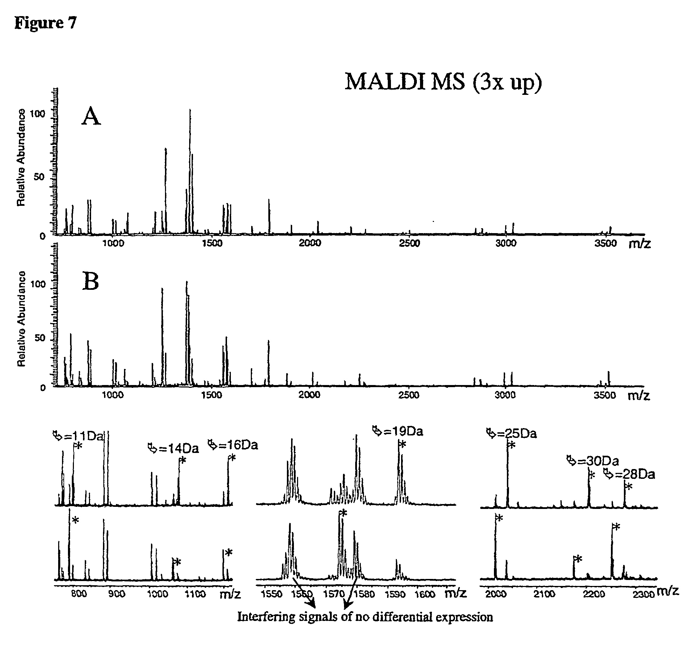

Figure 7. MALDI TOF detection of tryptic digests of an inverse ¹⁵N-labeled two-protein system with PTP protein 3-fold up-regulated in the "treated". (A): ¹⁴N-control- ¹⁵N-"treated" sample; (B): ¹⁵N-control- ¹⁴N-"treated" sample. The lower panels are the selective zoomed-in m/z regions.

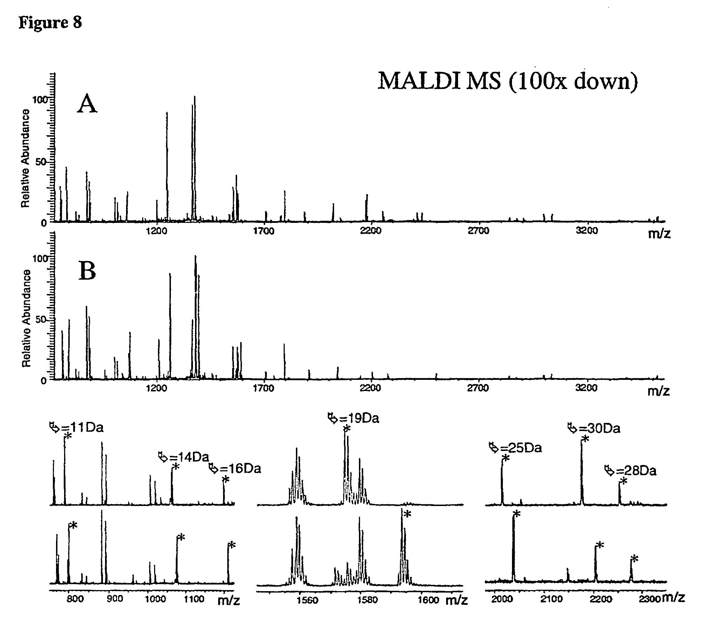

Figure 8. MALDI TOF detection of tryptic digests of an inverse ¹⁵N-labeled two-protein system with PTP protein 100-fold down-regulated in the "treated". (A): ¹⁴N-control - ¹⁵N-"treated" sample; (B): ¹⁵N-control- ¹⁴N-"treated" sample. The lower panels are the selective zoomed-in m/z regions.

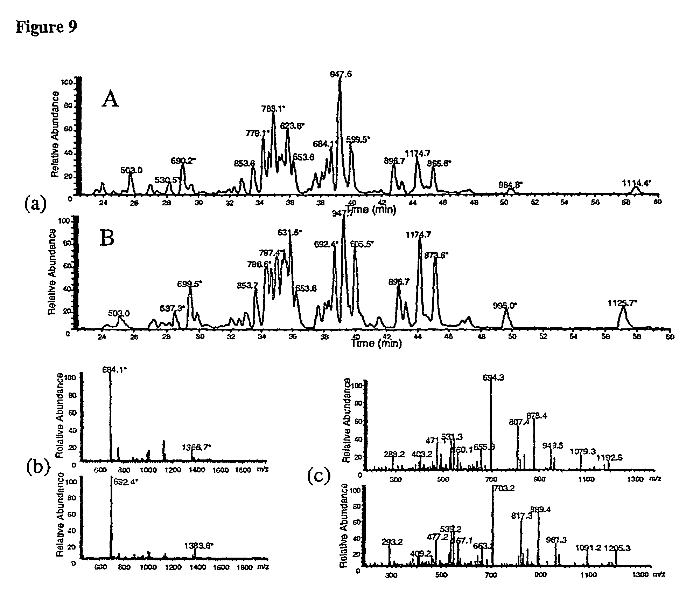

Figure 9. LC/MS-MS/MS detection of tryptic digests of an inverse ¹⁵N-labeled two-protein system with PTP protein 3-fold down-regulated in the "treated". (A): MS of the ¹⁴N-control- ¹⁵N-"treated" sample; (B): MS of the ¹⁵N-control- ¹⁴N-"treated" sample; (a) base-peak ion chromatograms of the two LC/MS-MS-MS runs; (b) MS spectra of a peptide in (a) displaying the inverse labeling pattern (mass shift); and (c) MS/MS spectra of the peptide in (b) (on the doubly charged ion). The PTP protein is exclusively identified from database searching using the MS/MS data of the ¹⁴N-peptide (upper (c)).

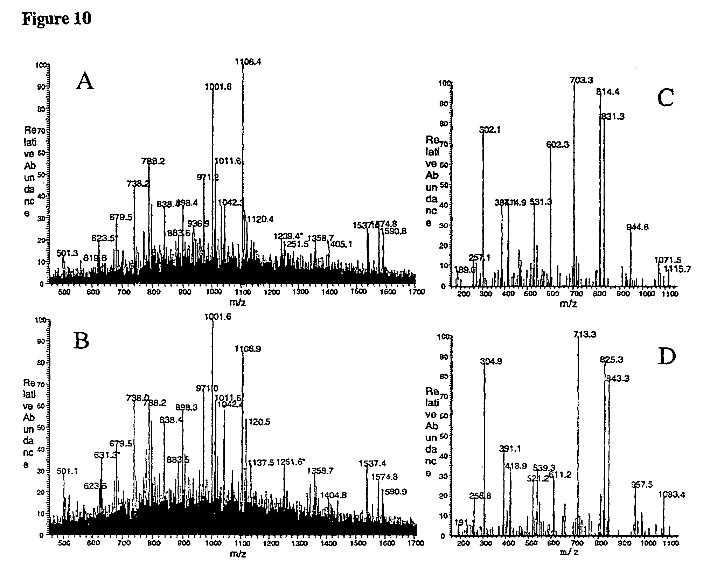

Figure 10. LC/MS-MS/MS detection of tryptic digests of an inverse ¹⁵N-labeled algal cell lysate spiked with PTP protein, with PTP 3-fold down-regulated in the "treated". (A): MS of the ¹⁴N-control- ¹⁵N-"treated" sample, averaged spectrum over a 3-min LC/MS window; (B): MS of the ¹⁵N-control- ¹⁴N-"treated" sample, averaged spectrum over a 3-min window; (C): MS/MS of the peptide in (A) m/z 623.5; and (D): MS/MS of the peptide in (B) m/z 631.3; where ¹⁴N-control is a 0.05 mg ¹³C-algal protein spiked with 10 pmol of PTP-1B; ¹⁵N-control is a 0.05 mg ¹³C- ¹⁵N-algal protein spiked with 10 pmol of ¹⁵N-PTP; ¹⁴N-"treated" is a 0.05 mg ¹³C-Algal protein spiked with 0.3 pmol of PTP-1B; and ¹⁵N-"treated" is a 0.05 mg ¹³C- ¹⁵N-algal protein spiked with 0.3 pmol of ¹⁵N-PTP. Mass shifts or inverse labeling pattern between (A) and (B) were observed on the marked ions (*). The inverse labeling or differential expression is further verified/confirmed in the MS/MS spectra by their similar fragmentation pattern. PTP-1B protein is exclusively identified from database searching using MS/MS data of the ¹⁴N-peptide (C).

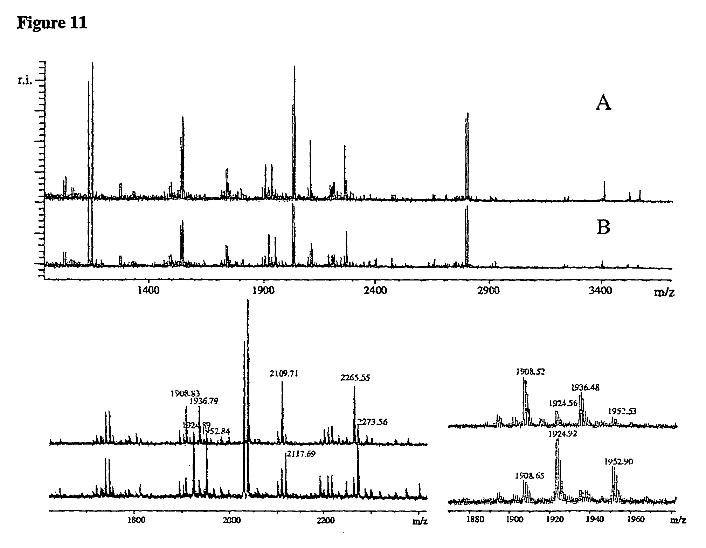

Figure 11. MALDI TOF detection of tryptic digests of an inverse ICAT-labeled six-protein system. (A): D₀-control - D₈-"treated" sample; (B): D₈-control - D₀-"treated" sample. The lower panels are the selective zoomed-in m/z regions. The mass shifts or D₀-/D₈-intensity ratio reversal indicates differential expression of proteins.

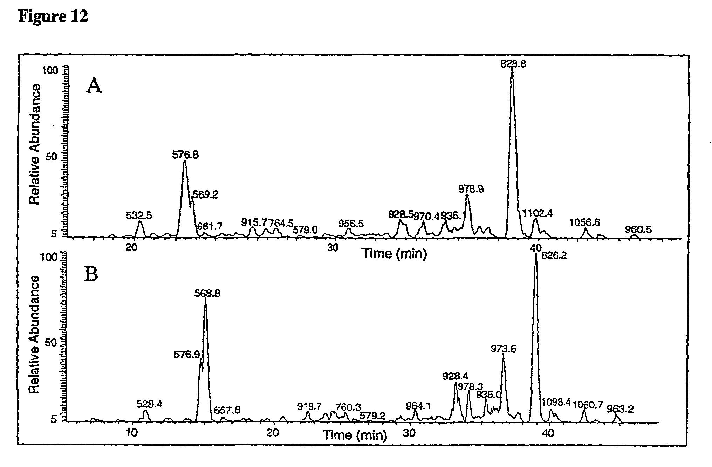

Figure 12. LC/MS detection of tryptic digests of an inverse ICAT-labeled six-protein system. (A): Base-peak ion chromatogram of the D₀-control - D₈-"treated" sample; (B): Base-peak ion chromatogram of the D₈-control - D₀-"treated" sample. Signals of the characteristic inverse labeling pattern of mass shifts are clearly detected. The differentially expressed proteins are quickly identified using their MS data.

### DESCRIPTION OF THE INVENTION

The term "differentially expressed" with respect to protein(s) refers to quantitative changes in expression level as well as qualitative changes such as covalent changes, e.g., post-translational modifications such as protein phosphorylation, protein glycosylation, protein acetylation and protein processing of the C- or N-terminal of a protein.

The term "sample" as used herein, is used in its broadest sense. Suitable samples include, but are not limited to, cell homogenates; cell fractions; tissue homogenates; biological fluids such as blood, urine, and cerebrospinal fluid; tears; feces; saliva; and lavage fluids such as lung or peritoneal lavages.

The term "stable isotope" refers to a non-radioactive isotopic form of an element.

The term "radioactive isotope" refers to an isotopic form of an element that exhibits radioactivity, i.e., the property of some nuclei of spontaneously emitting gamma rays or subatomic particles (e.g., alpha and beta rays).

The term "isotopically light protein labeling reagent" refers to a protein labeling reagent incorporating a light form of an element, e.g., H, ¹²C, ¹⁴N, ¹⁶O or ³²S.

The term "isotopically heavy protein labeling reagent" refers to a protein labeling reagent incorporating a heavy form of an element, e.g., ²H, ¹³C, ¹⁵N, ¹⁷O, ¹⁸O or ³⁴S. Isotopically light and isotopically heavy protein labeling reagents are also referred herein as unlabeled and labeled reagents, respectively.

The term "inverse labeling pattern" means a qualitative mass shift or an isotope peak intensity ratio reversal, i.e., from the heavy-labeled signal being stronger to the light-labeled signal being stronger (or vice versa), detected between the two inverse labeled mixtures.

The present invention relates to a novel procedure of performing protein labeling for comparative proteomics known as inverse labeling, which allows for the rapid identification of marker or target proteins, those in which expression levels have significantly changed upon a perturbation or those in which covalent changes have occurred upon a perturbation, e.g., as a result of either a disease state or drug treatment, contact with a potentially toxic material, or change in environment (e.g., nutrient level, temperature, passage of time). The rapid identification of differentially expressed proteins can be applied toward the revealment of new disease mechanisms, the elucidation of drug-action mechanisms and the study of drug toxicity. The method involves performing two converse collaborative labeling experiments in parallel on two different samples each containing a population of proteins. The two different samples are designated as the reference and experimental samples. These samples can differ in cell type, tissue type, organelle type, physiological state, disease state, developmental stage, environmental or nutritional conditions, chemical or physical stimuli or periods of time. For example, the reference and experimental samples can represent normal cells and cancerous cells, respectively; treatment without and with a drug, respectively, and the like.

The method comprises providing two equal protein pools from each of the reference and experimental samples. Each protein pool is then labeled with a protein labeling reagent, which is substantially chemically identical, except that it is distinguished in mass by incorporating either a heavy or light isotope. The isotope can be a stable isotope or a radioactive isotope. Incorporation of a stable isotope into the protein labeling reagent is preferred because it is stable over time thereby minimizing variations due to handling and thus provides more accurate quantitative measurements and is more environmentally safe than a radioactive isotope.

With respect to labeling of the protein pools, one protein pool from each of the reference and experimental samples is labeled with an isotopically heavy protein labeling reagent to provide an isotopically heavy-labeled reference pool and an isotopically heavy-labeled experimental pool. The remaining pool from each of the reference and experimental samples is labeled with an isotopically light protein labeling reagent to provide an isotopically light-labeled reference pool and an isotopically light-labeled experimental pool.

The protein labeling reagent can be any suitable reagent utilized to label proteins. The isotope is included in the reagent and thus is incorporated into the proteins. The labeling may be achieved chemically, metabolically, proteolytically or other suitable means to incorporate isotope into the proteins.

In one embodiment, the protein labeling reagent can be a reagent that contains a group that reacts with a particular functional group of a protein, Le., chemical labeling of the protein. Examples of reactive groups of protein labeling reagents include those that react with sulfhydryl groups, amino groups, carboxylic acid groups, ester groups, phosphate groups, aldehyde and ketone groups and the like. Examples of thiol reactive groups include, but are not limited to, nitriles, sulfonated alkyl or aryl thiols, maleimide, epoxides and alpha-haloacyl groups. Examples of amino reactive groups include, but are not limited to, isocyanates, isothiocyanates, active esters, e.g., tetrafluorophenylesters and N-hydroxylsuccinimidyl esters, sulfonyl halides, acid anhydrides and acid halides. Examples of carboxylic acid reactive groups include, but are not limited to, amines or alcohols in the presence of a coupling agent such as dicyclohexylcarbodiimide, or 2,3,5,6-tetrafluorophenyl trifluoracetate. Examples of ester reactive groups include, but are not limited to, amines which react with homoserine or lactone. Examples of phosphate reactive groups include, but are not limited to, chelated metal where the metal, e.g., Fe(III) or Ga(III) is chelated to nitrilotriacetic acid or iminodiacetic acid. Aldehyde or ketone reactive groups include, but are not limited to, amines and NaBH₄ or NaCNBH₄, such as described in *Chemical Reagents for Protein Modification* by R. Lundbald *(CRC Press* 1991).

One particularly useful type of protein labeling reagent is the affinity tag-containing reagent. Use of an affinity tag-containing reagent is particularly advantageous, in that specific classes of proteins, e.g., those containing phosphate groups, can be subjected to affinity purification, which can eliminate undesirable proteins thereby reducing the complexity of the protein pools and further enriching for particular classes of proteins. In addition, such affinity tag-containing reagents can also eliminate undesirable contaminants. that are incompatible or that would mask identification of specific proteins with mass spectrometry. For example, the above protein pools can be biotinylated with an isotopically heavy and isotopically light biotin-containing protein labeling reagent. Biotinylated-labeled proteins present in the protein pools can then be purified by biotin-avidin chromatography. The same principle can apply to peptides after proteolysis of the labeled protein mixtures to enrich particular classes of peptides or to reduce the mixture complexity, and thus potential interference on the identification of specific proteins with mass spectrometry.

The affinity tag for selective isolation of a protein or peptide modified with a protein labeling agent can be introduced at the same time as isotope incorporation, or, in a separate reaction prior to or post protein isotope labeling. In the case of a specific affinity tag reagent known as isotope-coded affinity tag (ICAT) reagent as described by Gygi et al, *supra,* the biotin affinity tag is part of the protein labeling reagent and is thus introduced at the same time as isotope labeling. Johnson et al. and Shaler et al., 2001, The 49^{th} ASMS Conference on Mass Spectrometry and Allied Topics, Chicago, Illinois; both describe affinity tags which are introduced prior to isotope labeling through amino acid-specific chemistry. After affinity enrichment of the tag-containing proteins/peptides, isotope labels can be introduced through a general modification scheme, such as N-terminal acylation, C-terminal esterification, or cysteine chemistry if a cleavable tag is employed as described, e.g., in Johnson et al, *supra.* Affinity tagging can also occur post isotope labeling. Included in such examples is the use of cysteine-specific biotinylation reagent to react and pool out cysteine-containing proteins/peptides after a general labeling procedure is performed such as N-terminal acylation, C-terminal esterification, or other non-chemical labeling methods such as metabolic ¹⁵N-labeling as described, e.g., in Conrads et al., Anal. Chem. 2001,73:2132-2139.

An example of a specific affinity tag-containing protein labeling reagent that has been used to label proteins derived from different samples for study of protein differential expression is the ICAT reagent as described, e.g., in Gygi et al., *supra*; and WO 00/11208. The structure of an ICAT reagent consists of three functional elements: 1) a biotin affinity tag, 2) a linker incorporating either H or ²H and 3) a protein reactive group, e.g., a sulfhydryl reactive group. In the ICAT method, the side chains of amino acid residues, e.g., cysteinyl residues, in a reduced protein sample are modified with the isotopically light form of the ICAT reagent. The same groups in a second protein sample are modified with the isotopically heavy form of the ICAT reagent. The two-labeled protein samples are combined and then proteolyzed to provide peptide fragments, some of which are labeled. The labeled (cysteine-containing) peptides are isolated by avidin affinity chromatography and then separated and analyzed by LC-MS/MS. An example of an ICAT reagent is biotinyliodoacetylamidyl-4,7,10 trioxatridecanediamine which consists of a biotin group for affinity purification, a chemically inert spacer which can be isotopically-labeled with stable isotopes for mass spectral analysis and an iodoacetamidyl group for reaction with sulfhydryl groups on proteins as described, e.g., in WO 00/11208. Similar strategies can be applied to the use of other reagents that contain different reactive groups for proteins.

In another embodiment, the protein labeling reagent can be a reagent that is able to be incorporated into the protein, e.g., by metabolic labeling of the protein pools. For example, the protein pools from the reference and experimental samples can represent different types of cells that are cultured in a culture medium containing an isotopically heavy or light-labeled assimilable source including, but not limited to, ammonium salts (e.g., ammonium chloride), glucose, or water, or one or more isotopically heavy- or light-labeled amino acids, e.g., cysteine, methionine, lysine, etc., to provide labeled proteins incorporating the heavy or light isotope, such as ¹⁵N and ¹⁴N, ¹³C and ¹²C, ²H and H, or ³⁵S and ³²S, respectively.

In a particularly useful embodiment, proteins are labeled as a direct result of proteolysis that is performed with the protein labeling reagent, ¹⁸O- and ¹⁶O-labeled water, as described e.g., in Rose et al., *Biochem. J.* 1983, 215:273-277; and Rose et al., *Biochem. J.* 1988, 250:253-259 and as set forth in more detail below.

Once labeling of the pools is completed, the isotopically light-labeled reference pool is combined with the isotopically heavy-labeled experimental pool to provide a first mixture. The isotopically heavy-labeled reference pool is then combined with the isotopically light-labeled experimental pool to provide a second mixture. Accordingly, in the first mixture, the isotopically heavy-labeled proteins are derived from the experimental pool, whereas in the second mixture the isotopically heavy-labeled proteins are derived from the reference pool. Through isotopic labeling, the identical protein in the reference and experimental samples is distinguished by mass to allow their independent detection and quantitative comparison between two samples by suitable techniques, e.g., mass spectrometric techniques.

The proteins in the first and second mixtures are preferably enzymatically or chemically cleaved into peptides by utilizing proteases, e.g., trypsin; chemicals, e.g., cyanogen bromide; or dilute acids, e.g., hydrogen chloride. Preferably, the labeled proteins are digested with trypsin. Typical trypsin:protein ratios (wt:wt) that are added to each protein solution range from about 1:200 to about 1:20. Digestion is allowed to proceed at about 37°C for about 2 to about 30 hours. Digestion of the proteins into peptides can also be carried out prior to or during labeling of each of the protein pools of the reference and experimental samples as is described in more detail below. The digestion step can be eliminated when analyzing small proteins.

The digested labeled peptides or labeled proteins from the first and second mixtures are then detected by any suitable technique capable of detecting the difference in mass between the isotopically labeled peptide or labeled protein derived from the reference and experimental samples. Preferably, the digested labeled peptides or labeled proteins are separated and subsequently analyzed by well known fractionation techniques as described below coupled with MS techniques which are well known in the art. While a number of MS and tandem MS (MS/MS) techniques are available and may be used to detect the peptides, Matrix Assisted Laser Desorption Ionization MS (MALDI/MS) and Electrospray ionization MS are preferred. The quantitative comparison of the separated labeled peptides or separated labeled proteins are reflected by the relative signal intensities for peptide or protein ions having the identical sequence that are labeled with the isotopically heavy and light labeled protein reagent. The chemically identical peptide or protein pairs are easily visualized during a mass spectrometric scan because they coelute or closely elute by chromatography and they differ in mass. If expression of a protein has been up or down regulated, i.e., a true shift in signal intensities of the light isotope and heavy isotope is observed in the first mixture, the inverse should be observed in analyzing the second mixture due to inverse labeling. If expression of a protein remains unchanged following a perturbation, there will be no significant difference in the labeling pattern between the first and second mixtures. Accordingly with inverse labeling, instead of quantitatively calculating the ratio of the isotopically light to isotopically heavy signals for every peptide as is carried out in prior art isotopic labeling methods for identifying the differentially expressed proteins, two data sets are readily compared to quickly identify peptides of such qualitative changes that are indicative of differentially expressed proteins.

Selective mass spectrometric detection may also be used to selectively detect a particular group of peptides after a general labeling scheme, such as by precursor ion scanning for the detection of phosphopeptides or glycopeptides as described, e.g., in Wilm, et al., Anal. Chem. 1996, 68: 527.

The sequence of one or more labeled small proteins or labeled peptides is determined by standard techniques, e.g., tandem mass spectrometry (MS/MS) or post source decay (PSD). At least one of the peptide sequences derived from a differentially expressed protein will be indicative of that protein and its presence in the reference and experimental samples. In addition, peptide fingerprint data can be generated by MS. Subsequently, data generated by MS of peptide fingerprints or peptide sequence information can be used to search a protein database for protein identification.

In a particularly preferred embodiment of the present method as exemplified below, protein pools of the reference and experimental samples are proteolyzed using trypsin prior to or at the same time of labeling with ¹⁸O- and ¹⁶O-water. One ¹⁸O-atom and one ¹⁶O-atom is incorporated into the newly formed carboxy terminus as a consequence of hydrolysis during proteolysis. An additional ¹⁸O and ¹⁶O may be incorporated into the terminal carboxy group through a mechanism of protease-catalyzed exchange as described, e.g., in Rose et al., 1988, *supra.* Thus, following digestion by trypsin all of the resulting peptides except for C-terminal peptides that lack Lys or Arg at the C-terminus are labeled with either one or two ¹⁸O- and ¹⁶O-atoms at the C-terminus (mostly two if enough time is allowed for exchange). Mainly for the purpose of conserving the expensive ¹⁸O-water, both during-proteolysis and post-proteolysis incorporation of ¹⁸O-labels have been explored. According to previous studies, ¹⁸O-labels may be incorporated into peptides at the C-terminal carboxy group through protease-catalyzed exchange. (See, e.g., Rose et al., 1988, *supra;* and Schnolzer et al., *Electrophoresis* 1996, 17:945-953.) This is confirmed by the observation that the majority of the non-C-terminal peptides are found to have incorporated more than one ¹⁸O-atom when a protein is digested in ¹⁸O-water. By adding a very small volume of ¹⁸O-water (∼10 µl) to a completely dried peptide mixture post-proteolysis (with or without additional trypsin) and allowing the exchange to occur at room temperature for 5-12 hours, the same level of ¹⁸O-incorporation is achieved as that of during-proteolysis labeling.

The post-proteolysis labeling can be very advantageous when dealing with proteins or protein mixtures for which reduction in volume is problematic. By doing post-proteolysis labeling, digestion can be carried out in the normal way in a regular water buffer, on cell lysate, or on membrane proteins, without worrying about protein precipitation during concentration or the use of a large quantity of the expensive ¹⁸O-water to reach an overwhelming ¹⁸O-enviromment for labeling. Once proteins are proteolyzed to peptides, concentration and precipitation is normally less of a problem, and the labeling process via protease-catalyzed exchange can be carried out using a very small amount of ¹⁸O-water. Another area where post-proteolysis labeling may prove to be very useful is in the performance of ¹⁸O-labeling experiments on gel-separated proteins via in-gel digestion. By carrying out ¹⁸O-labeling post-proteolysis, the amount of ¹⁸O-water required is substantially reduced, since the labeling is performed on the dried, extracted peptides. In contrast, the labeling will be performed on gels for during-proteolysis labeling where enough ¹⁸O-water has to be used to cover all swollen gel pieces.

Theoretical calculations on peptides up to 3,000 Da in size indicate that a change of 2.5-fold or higher in protein expression is likely required to achieve a clear and reliable observation of the characteristic ¹⁶O-/¹⁸O-intensity reversal or mass shift on the peptides between the two experiments. Accordingly, in the two model systems exemplified below, a value of three-fold is chosen for use. In both cases, peptides of the characteristic inverse labeling pattern are clearly detected, and with the data, the expected proteins are exclusively identified from the databases. In reality, protein differential expression, typically with a two-fold or greater difference in expression levels, is considered to be statistical significant. In a typical proteomic analysis involving disease and normal mammalian material, 50-300 such unique or differentially expressed proteins may be identified as described, e.g., in Page et al., *Drug Discovery Today* 1999, 4:55-62. A cut-off value such as five-fold or greater in protein changes may be applied to focus on the most important proteins.

Additional fractionation schemes at the protein or peptide level may be required in order to reduce the complexity of the proteins in the reference and experimental samples, and complexity of protein mixtures or peptide mixtures that reach the mass spectrometer to reduce the chances of interference of separated peptides or small proteins and thus clear detection of the inverse labeling pattern and the identification of the proteins. Conventional fractionation techniques for reducing the complexity of protein mixtures include, but are not limited to, ammonium sulfate precipitation, isoelectric focusing, size exclusion chromatography, ion exchange chromatography, adsorption chromatography, reverse phase chromatography, affinity chromatography, ultrafiltration, immunoprecipitation and combinations thereof. Conventional fractionation techniques for reducing the complexity of peptide mixtures include, but are not limited to, size exclusion chromatography, ion exchange chromatography, adsorption chromatography, reverse phase chromatography, affinity chromatography, immunoprecipitation and combinations thereof. For example, generic affinity procedures can be applied after a general labeling scheme to isolate a particular class of peptides. Such examples include the use of immobilized metal affinity columns (IMAC) to enrich phosphopeptides, and the use of Con A beads for isolating glycosylated peptides as described, e.g., in Chakraborty et al; and Regnier, 2001, The 49^{th} ASMS Conference on Mass Spectrometry and Allied Topics, Chicago, Illinois.

The inverse labeling method is schematically illustrated in Figure 1. In this method, each of the two protein pools that are to be differentially compared (e.g., a control vs. a disease state) is divided into two equal portions. One portion from each of the two pools is labeled with, e.g., a reagent containing a heavy isotope, e.g., ¹⁸O, by the above method while the remaining portion is not labeled, i.e., labeled with a light isotope, e.g., ¹⁶O (Figure 1). Then a portion from the control and a portion from the perturbed are combined so that in the first experiment the labeled proteins are derived from the perturbed pool and, in the second experiment, the labeled proteins are derived from the control pool. If expression of a protein has been significantly up or down regulated by the perturbation (i.e., a true shift in signal intensities of ¹⁶O and ¹⁸O is observed in one analysis), the inverse should be observed in the analysis, of the other sample due to the inverse labeling.

As depicted in Figure 1, the rapid identification of differentially expressed proteins is achieved via quick identification of peptides derived from those proteins that exhibit the characteristic inverse labeling pattern. For most proteins, their expression level remains unchanged following perturbation which is reflected by a similar abundance profile of pool 1 and pool 2. Therefore, there will be no significant difference in the labeling pattern between the two inverse labeling experiments (i.e:, similar abundance of ¹⁶O- and ¹⁸O-signals in both experiments), and these signals can be subtracted out, in principle, by the comparative analysis of the two data sets. The C-terminal peptides without ¹⁸O-labeling are subtracted out as well. For a protein in which the level of expression has been significantly up or down regulated by the perturbation, changes in the ¹⁶O- and ¹⁸O-signal intensities will be observed. When the control is not labeled and the perturbed is ¹⁸O-labeled, the ¹⁸O-signal will be of greater intensity if the protein is up-regulated; conversely, the ¹⁶O-signal will be stronger if a down-regulation has occurred. The inverse will be observed in the second analysis where the labeling is reversed. Depending on the direction of the intensity-ratio reversal between the two analyses, the direction of differential expression of the protein (i.e., up-regulation or down-regulation) can be determined. For example, if a protein is substantially up-regulated by a disease state in pool 2 in comparison to the control pool 1, and when the disease sample is ¹⁸O-labeled, higher intensities of the ¹⁸O-signals for all peptides from this protein will be observed except for the C-terminal peptide. When the labeling is inverted in the second experiment in which the control pool is ¹⁸O-labeled while the disease pool is not labeled, the ¹⁶O-signals will be stronger for those peptides. Thus, there is a 2/4 Da downward mass shift of the more intense isotopic ion between the two inverse labeling experiments (i.e., from ¹⁸O-signal in the first experiment to ¹⁶O-signal in the second experiment). In reality, for peptides of higher masses (e.g., 1300 Da or larger), the mass shift between the two analyses on the most intense ion may be detected as 1/3 Da rather than 2/4 Da due to the ¹³C-interference when the protein differential expression is not sufficiently significant to omit the ¹³C-effect. The mass shift of the most intense isotopic ion here reflects the intensity-ratio reversal. With this procedure, instead of quantitatively calculating the ratio of the ¹⁶O- to ¹⁸O-signals for every peptide, one only needs to compare the two data sets and identify peptides of the characteristic mass shift, which can be achieved rapidly and potentially automatically. The direction of the shift implicates either an up- or down-regulation of the effected proteins.

In identifying differentially expressed proteins, the inverse labeling approach using any suitable labeling method overcomes difficulties inherent in other prior art approaches that utilize mass spectrometry as described below.

Any statistically significant change in protein expression level should display an inverse labeling pattern in the inverse labeling experiments. For metabolic ¹⁵N-labeling, the mass increase upon labeling is a variable depending on the sequence of the peptides (with a range of about 1.0-1.5% of the peptide MW averaged at about 1.2%). The variable or unpredictable mass difference makes it extremely difficult to correlate peptide isotope pairs using a conventional mass spectrometer if the spectra are highly complexed. The use of ultrahigh resolution FT ICR (fourier transform ion cyclotron resonance) MS has been suggested for measurement of high accuracy to obtain accurate mass differences between peaks and therefore assign peptide isotopic pairs with high confidence. Another possible but impractical solution is through the use of tandem MS. The isotopic pair of peptides should possess a similar fragmentation pattern and can thus be correlated using their MS/MS data. In the application of the inverse labeling method, what one looks for is the qualitative mass shifts, not isotopic pattern, nor accurate mass shifts. Therefore there is no stringent requirement on resolving power of the MS instruments. A mass shift is readily recognized even though the isotopic peaks may not be fully resolved for peptide ions of higher charge states using a standard mass spectrometer of unit resolution. The observation/conclusion is further supported by the similar fragmentation pattern of the MS/MS data, which is obtained for the logical subsequent step in the process of achieving the identification of the proteins. Redundant work would have to be carried out using the other solutions, either by measuring accurate mass differences of multiple signal pairs to select a best-fit pair, or by performing MS/MS on all signals and find a correlated pair based on similarity of fragmentation pattern. The approach of using MS/MS fragmentation pattern for achieving correlation of isotope pairs not only requires tremendous amount of instrument time to acquire the data, it also demands major effort in data handling (impossible to do manually). Difficulties would always be present when an isotope signal is too weak for an accurate mass measurement or getting a useful MS/MS data. When inverse labeling is not performed, ambiguity is a real concern when unpaired (isotope) signals are detected in the cases of protein covalent changes or extreme changes in expression. Unpaired signals detected can be confused as unlabeled peptides/proteins or chemical backgrounds. A qualitative shift will be observed with inverse labeling if a true change has occurred to a protein quantitatively or qualitatively. With the inverse labeling approach, one can use any mass spectrometer of standard unit resolution, and acquire only the minimum, essential data to achieve the rapid identification of differentially expressed protein markers/targets without ambiguity. Relative quantitation of expression level, again only on the differentially expressed proteins (or proteins of interest) can be performed afterwards if desired.

The following examples serve to illustrate the invention but do not to limit the scope thereof in any way.

### EXAMPLES

### Materials

¹⁸O-water (95% atom) is purchased from Isotec Inc. (Miamiburg, OH).

¹³C-algal protein extract and ¹³C-¹⁵N-algal protein extract are purchased from Isotec Inc. (Miamisburg, OH).

ICAT reagent (both light D₀ and heavy D₈) is purchased from Applied Biosystems (Cambridge, MA).

### Example 1

### Inverse ¹⁸O-Labeling Utilizing an Eight-Protein Model System

Commercial proteins of BSA, aldolase, carbonic anhydrase, β-casein, chicken albumin, apo-transferrin, β-lactoglobulin, and cytochrome C (Sigma) are used without further purification. The eight proteins are mixed at a molar ratio of 1:1:1:1:1:1:1:1 for the "control" and 0.3:3:1:1:1:1:1:1 for the "treated" pool. Two identical aliquots containing 10 pmol each of the unchanged components are taken from each pool and are dried using a Speedvac. The ¹⁸O-labeling is performed using two procedures, during proteolysis and post-proteolysis. For proteolysis labeling, one of the dried aliquots is reconstituted with 20 µl of regular water and the other with 20 µl of ¹⁸O-water, both containing 50 mM ammonium bicarbonate. Trypsin (Modified, Promega) at a 1:100 trypsin-to-protein ratio (wt:wt) is added to each solution and digestion is allowed to proceed at 37°C for ∼20 hrs. For the post-proteolysis labeling, all trypsin digestions are performed in regular water-ammonium bicarbonate buffer at the same trypsin to protein ratio for ∼12 hrs. The resulting peptide mixtures are then taken to complete dryness with a Speedvac. 10 µl of ¹⁸O or regular water are added respectively to the dried peptide mixtures for post-proteolysis ¹⁸O-labeling. The process is allowed to proceed at room temperature for ∼12 hrs. Prior to analysis, for both during-proteolysis and post-proteolysis labeling, the ¹⁶O-control sample is mixed with the ¹⁸O-"treated" sample and the ¹⁸O-control sample is mixed with the ¹⁶O-"treated" sample. The same MS analysis is performed on both mixtures.

### Example 2

### Inverse ¹⁸O-Labelling Utilizing Whole Cell Lysate Spiked With PTP (Protein Tyrosine Phosphatase)

Approximately 5x10⁷ harvested CHO cells are lysed mechanically (freeze/thaw) using a buffer containing 10 mM Tris, 1 mM EDTA, pH 7.4. The resulting cell lysate of 2.5 ml at 0.4 mg/ml protein concentration is divided into four aliquots. Two are spiked with 10 pmol of PTP-1B protein (internally expressed, residue 1-298) (PTP10) and the other two with 30 pmol of PTP-1B (PTP30). Trypsin is added to each solution at a 1:100 (wt:wt) trypsin-to-total protein ratio to initiate the digestion. The proteolysis is allowed to proceed at 37°C for ∼12 hrs. The resulting solutions are centrifuged and the solid discarded. The solutions are then taken to complete dryness with a Speedvac. For both PTP10 and PTP30, one of the two identical aliquots is reconstituted with 10 µl of ¹⁸O-water, the other with 10 µl of regular water. The post-proteolysis ¹⁸O-incorporation is allowed to proceed at room temperature for ∼12 hrs. Prior to analysis, the ¹⁶O-PTP10 and ¹⁸O-PTP30 samples are mixed, and so are the ¹⁸O-PTP10 and ¹⁶O-PTP30 samples. Each mixture is diluted with 100 µl of mobile phase A (0.1% formic acid - 0.01% TFA in water) and filtered through a 0.4 µm Microcon filter. The filtrate is injected to LC/MS for analysis.

### Example 3

### LC/MS and LC/MS/MS Peptide Analysis of Inverse ¹⁸O-Labeled Peptide Mixtures

MS analysis of the inverse ¹⁸O-labeled peptide mixtures is carried out through LC-ESI MS using a Finnigan LCQ ion trap mass spectrometer. A 1.0 x 150 mm Vydac C18 column is employed for on-line peptide separation with a gradient of 2-2-20-45-98-98% B at 0-2-10-65-66-70 min. The mobile phase A is 0.1% formic acid - 0.01% TFA in water and B is 0.1% formic acid - 0.01% TFA in acetonitrile. The flow rate is 50 µl/min. Post-LC column, the flow is split 9:1 with about 5 µl/min going into MS and 45 µl/min being collected for later use. LCQ ion trap mass spectrometer is operated at a data-dependent mode automatically performing MS/MS on the most intense ion of each scan when the signal intensity exceeds a pre-set threshold. When needed, the collected samples are concentrated and re-analyzed to obtain MS/MS data that are not collected automatically in the first run for the peptides of interest. The relative collision energy is set at 45%. Under this condition, most peptides fragment effectively in our experience. An 8-Da window for precursor ion selection is employed.

### Example 4

### MALDI TOF MS Peptide Analysis of Inverse ¹⁸O-Labeled Peptide Mixtures

The mixture samples are simply diluted 1:3 to 1:5 using the MALDI matrix solution (saturated α-cyano-4-hydroxy cinnamic acid in 50% acetonitrile - 0.1% TFA) and ∼1 µl of the final solution (containing about 500 finol each based on the unchanged components for the eight-protein system) are loaded onto MALDI target for analysis. The analysis is performed on a Bruker REFLEX III MALDI TOP mass spectrometer operated in the reflectron mode with delayed ion extraction. When applicable, post source decay (PSD) is also performed on the peptide ions of interest.

### Example 5

### Database Search of Inverse ¹⁸O-Labeled Peptides

Search software PROWL (Proteometrics, New York, NY) and MASCOT (Matrix Science, London, UK) are used to search protein databases to identify proteins using peptide fingerprints, MS/MS fragments, and processed PSD spectra. For searches using peptide fingerprint information, peptide ions exhibiting the inverse labeling pattern or mass shift of 2 or 4 Da on the most abundant isotopic ion between the two inverse labeling experiments are sorted out based on the direction of mass shift (up or down). Each list is used separately for a database search to identify the proteins. For searches using peptide sequence information, the MS/MS spectra of a peptide from the two inverse labeling experiments are compared and Y ions with a mass shift of 2 or 4 Da are identified. These ions are used alone or in combination with B ions to search protein databases to obtain identification of the proteins. An iterative search combining the data of the peptide map and MS/MS is also performed. Any ions that demonstrate a clear inverse labeling pattern in the map and are supported by mass shifts of fragment ions in MS/MS data are identified first using their MS/MS fragments/sequence tags. The peptides associated with the identified proteins are then removed from the list and a second round search is initiated using the masses of the remaining peptides. For the ions for which no convincing conclusion could be made, a second analysis using the collected sample is performed to obtain MS/MS data on them. The resulting data are used in the same manner to search the databases for protein identification.

### Example 6

### MS Analysis of Inverse ¹⁸O-Labeling Method Using the Eight-Protein Model System

The inverse ¹⁸O-labeling and MS analysis are performed in a similar fashion as shown in Figure 1 on the eight-protein model system where BSA is "down-regulated" by 3-fold and aldolase "up-regulated" by 3-fold. When analyzed using an LCQ with on-line RP LC, a clear inverse labeling pattern or a 2/4 Da mass shift is observed for a number of peptides (Figures 2-3 (A, B)). Following data analysis, two lists of peptide masses that are based on the direction of the mass shift are quickly formed. When each is used separately to search the database, aldolase is exclusively identified using the list of 2/4 Da downward shift, corresponding to an up-regulation of protein expression, while BSA is identified using the list of upward mass shift, which corresponds to a down-regulation in protein expression. MS/MS spectra are obtained automatically at data dependent mode on a few of the peptides. An iterative search scheme is also applied, using the combined mass list of all that shifted, regardless of the direction of the shift. Once a protein is identified with high confidence (aldolase in this case), with either the mass list or an MS/MS spectrum, the related peptides of the protein are removed from the mass list. A second search is then performed on the remaining list to identify the second most prominent protein (BSA in this case). As a consequence of inverse labeling, very rich information is embedded in the MS/MS data. First, since the label is incorporated at the C-terminus of each peptide, Y ions in an MS/MS spectrum are the fragments carrying the label and exhibit the characteristic inverse labeling pattern for proteins that are differentially expressed. As shown in Figures 2-3 (C, D), for proteins whose "expression level" is significantly altered by "perturbation", the inverse labeling pattern or a 2/4 Da mass shift observed at the molecular ion level on the peptides is passed on to the Y ions in the MS/MS spectra. The observation of the characteristic inverse labeling pattern on the fragment ions in the MS/MS spectra provides further verification and confirmation of protein differential expression. Since most peptide fragments carry fewer charges than the parent molecule (mostly singly charged in the figures shown in this paper), the mass shift is more prominent and thus is easier to recognize compared to that from their multiply charged precursor ion. Secondly, the inverse labeling pattern that is reflected in Y ions in the MS/MS spectra, in turn, offers a very convenient way to identify Y ions and B ions for the interpretation of an MS/MS spectrum. The fragments with mass shifts are Y and Y-related ions and the ones without mass shift are B or B-related ions. Although interpretation is not required to search the databases using MS/MS data, added specificity helps to increase efficiency and accuracy of protein identification via database search. Both BSA and aldolase are positively identified using the MS/MS data and the Y/B ion assignments (Figures 2-3). In fact, all expected proteins are identified using the MS/MS data and the Y/B ion assignments (Figures 2-3 and 5-6). These advantages are of more importance when one deals with novel proteins where *de novo* sequencing is required. The ability to assign Y and B ions greatly facilitates "read out" of the sequence from an MS/MS spectrum. Although accurate quantitation of protein expression is not the intended use of the method, the information is available in both MS and MS/MS data, if one desires to perform the task (i.e., signal intensities of ¹⁶O to ¹⁸O after correction of the natural ¹³C-isotopic contribution). MALDI TOF MS performed directly on the mixture without any separation results in a peptide-map spectrum that shows severe overlap, which makes data interpretation difficult (Figure 4 (A, B)). Nonetheless, the inverse labeling pattern can still be observed for a number of ions (Figure 4 (C, D)). PSD is carried out on a few of the ions and the proteins are able to be identified using the PSD data (Figure 5).

### Example 7

### MS Analysis of Inverse ¹⁸O-Labeling Using PTP-Spiked Cell Lysate System

On the whole cell lysate system where PTP-1B protein is spiked in at two different levels with the intention to mimic a complex protein mixture system, a lot of peptide signals with good signal intensities are detected (data not shown). Even with on-line LC separation, severe overlapping is expected and, indeed, observed. Nonetheless, when the two sets of data from inverse labeling are analyzed and compared, a few ions are identified with the characteristic inverse labeling pattern, primarily with a 4 Da shift (Figure 6 (A, B)). The split and collected samples are subjected to a second round of analysis to obtain their MS/MS data. The MS/MS data with Y ions exhibiting the inverse labeling pattern of a 4 Da shift between the two parallel experiments further verify/ confirm the mass shift observed on the precursor peptides and, thus, the differential expression of the protein (Figure 6 (C, D)). A database search using the readily recognized Y ions of mass shift leads to the conclusive identification of the protein as human PTP-1B. In this particular case with whole cell lysate, as expected, MALDI MS peptide mapping does not provide much useful information due to severe overlapping of the peptide signals (data not shown).

Unlike metabolic labeling of proteins during cell culture (¹³C/¹⁵N²H), this approach doesn't require any special skill and/or facility. Also, analysis of tissue proteins and identification of marker/target proteins from tissues can be readily performed. Unlike chemical labeling, this method does not involve additional reaction/work-up steps. Thus, it avoids potential sample loss associated with the additional steps. Another pitfall associated with the residue-specific chemical labeling, namely, high likelihood of losing post-translational modification information, is also avoided. Because two collaborative analyses are performed with the inverse labeling method, signals of no isotopic counterpart detection either due to extreme changes in expression level and the dynamic range limitation of MS detection or covalent modifications of proteins can be identified without ambiguity.

### Example 8

### Inverse ¹⁵N-Labeling Utilizing a Two-Protein Model System

Regular and ¹⁵N-labeled PTP protein (1-298) and regular and ¹⁵N-labeled HtrA protein (161-373) are internally prepared using standard culture conditions with the ¹⁵N-labeled materials being produced by fermentation in ¹⁵N-enriched culture media. The authenticity of the proteins and the level of isotope incorporation are assessed by MS on the final protein products. The labeling yield is better than 90% for both proteins according to MS results. The two-protein model systems are made by mixing together the two individual proteins, PTP and HtrA, with the regular ¹⁴N-mixture being the mixture of the two ¹⁴N-proteins, and the ¹⁵N-mixture as the mixture of the two ¹⁵N-labeled proteins. The "control" is a mixture of two proteins at a molar ratio of 1:1. The "treated" or "altered state" materials are made to mimic four different levels of "protein differential expression" for PTP protein while the level of "expression" of HtrA remains unchanged. The molar ratios of PTP:HtrA for the four ''treated'' mixtures are 3:1, 100:1, 0.3:1, 0.01:1 mimicking a 3-fold and a 100-fold up-regulation and a 3-fold and a 100-fold down-regulation, respectively. The regular ¹⁴N-mixtures and the labeled ¹⁵N-mixtures are made in the same manner. To perform the inverse labeling experiments, an aliquot of ¹⁴N-control is mixed with an aliquot of ¹⁵N-"treated" (each containing the same amount of HtrA protein) while the inverse labeling is achieved by combining the ¹⁵N-control with the ¹⁴N-"treated" in the same fashion. (Two inverse labeling mixtures are thus produced for each comparative proteomic experiment.) The same procedure is performed for all four "differential" levels. The subsequent trypsin digestion is carried out on all the mixtures at a 1:50 trypsin-to-protein ratio (wt:wt) (Modified trypsin from Promega, sequencing grade) at 37°C for ∼7 hrs in 50 mM ammonium bicarbonate buffer (the two proteins are known to readily digest under this condition without prior reduction and alkylation). MS analysis using both MALDI and electrospray LC/MS is performed on all peptide mixtures. Aliquots each containing 10 pmol of HtrA peptides are used for the LC/MS analysis.

### Example 9

### Inverse ¹⁵N-Labeling Utilizing Algal Cell Lysate Spiked With PTP Protein

A 1 ml solution containing 6M Guanidine HCl-50 mM Tris-50mM NaCl pH 7.4 is added to 10 mg each of a ¹³C-algal protein extract and a ¹³C-¹⁵N-algal protein extract. The mixtures are vortexed and sonicated for 40 min to solubilize the proteins. After centrifuge at 20,000 RPM for 20 min, the supernatants are taken out for further use. A large amount of insoluble is discarded. 10 mM DTT is added to the solutions and reduction reaction continues for 1 hr at 50°C. Cysteine alkylation is carried out by the addition of 40 mM iodoacetic acid sodium salt followed by shaking at room temperature in the dark for 1 hr. A Centricon filter of 1kDa MW cutoff is subsequently used to remove the excess reagents and to exchange the buffer to 50 mM ammonium bicarbonate. Protein concentration of the extracts is measured using the standard Bradford method. 10 pmol of regular PTP protein is spiked into an aliquot of ¹³C-algal protein extract containing about 0.05 mg of total protein to form the ¹⁴N-"control", and 10 pmol of ¹⁵N-PTP is spiked into an aliquot of ¹³C-¹⁵N-algal protein extract containing about 0.05 mg of total protein as the ¹⁵N-"control". As for the "treated", a 3-fold down-regulation is created by spiking 3 pmol of PTP into an identical aliquot of algal extract, and a 100-fold down-regulation is made by spiking 0.1 pmol PTP into another equal aliquot of algal extract. The ¹⁴N-material is the result of ¹⁴N-PTP being spiked into the aliquot of ¹³C-algal extract, and, the ¹⁵N-material is produced by spiking ¹⁵N-PTP into aliquot of ¹³C-¹⁵N-algal extract. The inverse labeling experiments proceed in the same way by combining aliquots of ¹⁴N-control with ¹⁵N-"treated", and ¹⁵N-control with ¹⁴N-"treated". Trypsin digestion on the four resulting inverse labeling mixtures (for two differential levels) is performed at a 1:100 trypsin-to-protein ratio (wt:wt) at 37°C for ∼16 hrs in 50 mM ammonium bicarbonate buffer. All digests are analyzed by electrospray LC/MS.

### Example 10

### MALDI TOF MS Peptide Analysis of The Inverse¹⁵N-Labeled Peptide Mixtures

All digest mixtures of the two-protein model systems are analyzed by MALDI TOP MS. The mixture samples are diluted 1:5 using the MALDI matrix solution (saturated α-cyano-4-hydroxy cinnamic acid in 50% acetonitrile - 0.1% TFA) and ∼1 µl of each of the final solutions (containing about 500 fmol of HtrA peptides) is loaded onto MALDI target for analysis. The analysis is performed on a Bruker REFLEX III MALDI TOF mass spectrometer operated in the reflection mode with delayed ion extraction.

### Example 11

### LC/MS And LC/MS/MS Peptide Analyses of The Inverse ¹⁵N-Labeled Peptide Mixtures

All digest mixtures of the two-protein model systems and those from the algal-spiking systems are analyzed by LC/MS-MS/MS. The analysis is carried out through electrospray LC/MS using a Finnigan LCQ ion trap mass spectrometer. A 1.0 x 150 mm Vydac C18 column is employed for on-line peptide separation. A gradient program of 2-20-45-98-89%% B at 0-10-65-66-70 min is used. Mobile phase A is 0.25% formic acid in water and mobile phase B is 0.25% formic acid in acetonitrile. The flow rate is 50 µl/min. After the elution from the LC column, the flow is split 9:1 with about 5 µl/min going into MS and 45 µl/min being collected for later use. The LCQ ion trap mass spectrometer is operated at a data-dependent mode, automatically performing MS/MS on the most intense ion of each scan when the signal intensity exceeds a pre-set threshold. When needed, the collected samples are concentrated and re-analyzed to obtain MS/MS data that are not collected automatically in the first run for the peptides of interest. The relative collision energy is set at 45% at which most peptides fragment effectively. A 5-Da window for precursor ion selection is employed.

### Example 12

### Database Search of The Inverse ¹⁵N-Labeled Peptides

Search software PROWL (Proteometrics, New York, NY) and MASCOT (Matrix Science, London, UK) are used to search the protein databases to identify proteins using peptide fingerprints, and MS/MS fragments. For searches using peptide fingerprint information, peptide ions exhibiting the inverse labeling pattern between the two inverse labeling experiments are sorted out based on the direction of mass shift (increasing or decreasing). Each list is used separately for a database search to identify the proteins. For searches using peptide sequence information, the MS/MS spectra of a peptide from the two inverse labeling experiments are compared and their correlation is further verified/confirmed by their similar fragmentation pattern. The MS/MS spectrum of the N¹⁴-peptide (lower in mass) is used to search databases, for protein identification.

### Example 13

### MS Analysis of Inverse ¹⁵N-Labeling Method Using the Two-Protein Model System

Direct MALDI analysis is successfully carried out on the mixtures of the two-protein model system. Off-line coupling of separation (such as with two-dimensional chromatography) with MALDI TOF MS on a digest of a complexed protein mixture (e.g., total cell lysate) can in each fraction resemble the situation demonstrated here. In contrast to the inverse labeling method, when the single-experiment approach is applied, even for the cases where protein differential expression is not so drastic that both isotope pairs are clearly detected (e.g., 3-fold change, Figure 7 (A)), correlation of isotopic pairs can still be difficult to achieve such as that shown in the m/z range of 1550-1600. However, by subtractive comparison of two MALDI spectra from an inverse labeling experiment (Figures 7-8 (A, B)), signal pairs from proteins of no significant differential expression can be subtracted out (such as those marked with arrows along the horizontal axis) and result in much simplified spectra for easier correlation. When protein differential expression is not too drastic (e.g., 1000-fold or less) and both isotope signals are detected, the reversal in signal intensity ratio is easily recognized to support the correlation (Figure 7 (A, B)). Mistakes are more likely to happen, if inverse labeling is not used, in correlating isotopic pairs when a more dramatic differential expression has occurred such that the weaker isotopic signals are not detected due to the dynamic range limitation in MS detection. Falling into the same category is covalent change of protein as a result of a perturbation where covalent modifications of proteins occur such as protein processing at terminus or post-translational modifications. The peptides bearing the covalent changes will be detected without the isotopic counterpart since the modifications are not present in the control state. Inverse labeling offers an easy solution to these problems. Although a 100-fold down regulation is not drastic enough for the weaker isotope signals to completely escape detection, it is a good example to demonstrate the benefits of the approach. As shown in Figure 8 (A, B), the inverse labeling pattern is readily recognized after the subtractive cleanup of signals from proteins of no significant differential expression. (Keeping in mind that the range of nitrogen atoms per peptide sequence should normally be larger than 1% of the peptide molecular weight and smaller than 1.5% of the peptide MW, and averaged at about 1.2% MW.) The digestion mixtures from the two-protein model systems are also analyzed by electrospray LC/MS (Figure 9 (a-c)). The data suggest that the isotopic pairs do not display any significant separation by reverse phase chromatography. A quick comparison of the two base-peak ion chromatograms from an inverse labeling experiment (Figure 9 (a)) leads to the rapid identification of the base-peak peptides of inverse labeling pattern (mass shifts) or from proteins of differential expression. Certainly, one has to process the MS data in order to identify other peptides of inverse labeling pattern that are in lower abundance and co-eluting with more abundant peptides. Once the peptide signals with inverse labeling pattern are identified, the MS/MS data that are acquired automatically in data-dependent mode of operation are analyzed. Their similar fragmentation pattern would verify/confirm the correlation of isotopic pairs and thus the correct conclusion on protein differential expression. The data are then used to search protein databases for protein identification (Figure 9 (c)). In this case, PTP-1B protein is readily identified from the database. In practice, when dealing with a complexed protein system, an iterative search scheme combining the data of ions with inverse labeling pattern from peptide map and MS/MS may be performed. Any ions that demonstrated a clear inverse labeling pattern in the map and are further supported by similar fragmentation patterns of MS/MS data are identified first using their MS/MS data (of ¹⁴N-ion or lower mass). The peptides associated with the identified proteins can then be removed from the peptide list and a second round search is initiated using the MS/MS data of the remaining peptides of inverse labeling pattern. For those ions of no MS/MS data automatically acquired, a second analysis is performed using the collected sample to obtain their MS/MS data. The data are then used in the same manner to search the databases for protein identification.

### Example 14

### MS Analysis of Inverse ¹⁵N-Labeling Method Using the Spiked Algal Cell Lysate System

To demonstrate the application of the approach in a more complexed mixture, PTP-1B protein, both non-labeled and ¹⁵N-labeled, are spiked into algal cell lysate - ¹³C and - ¹³C/¹⁵N, respectively, at different levels (3-fold and 100-fold down-regulation) to mimic protein differential expression. The inverse labeling experiment is then performed and the mixtures are analyzed by LC/MS-MS/MS. When two sets of data from each inverse labeling experiment are compared, a number of ions possessing the characteristic inverse labeling mass shifts are extracted (Figure 10 (A, B)). The split and collected samples are subjected to a second analysis to obtain MS/MS on the ions that exhibit the inverse labeling pattern. Their similar fragmentation patterns clearly validates the mass shift or inverse labeling pattern observed on the precursor peptides and, thus, the differential expression of the precursor protein (Figure 10 (C, D)). A database search using the MS/MS data of ¹⁴N-peptide leads to the exclusive identification of the human PTP-1B protein.

### Example 15

### Inverse ICAT Labeling Utilizing a Six-Protein Model System

Commercial proteins of BSA, aldolase, β-casein, apo-transferrin, β-lactoglobulin, and cytochrome C (Sigma) are used without further purification. The six proteins are mixed at a molar ratio of 1: 1: 1: 1: 1: for the "control" and 0.3:3:1:1:1:1 for the ''treated'' pool. The recommended protocol is followed. The protein mixtures of control and "treated" are first reduced and denatured. ICAT derivatization is then performed in the inverse labeling way (Figure 1), with half of each mixture reacting with D₀ -ICAT reagent and the remaining half reacting with D₈- ICAT reagent. The inverse labeling proceeds by mixing the D₀-control with the D₈-"treated", and the D₈-control with the D₀-"treated". Trypsin digestion is then performed on both mixtures at 1:50 (wt:wt) trypsin-to-protein ratio for ∼16 hrs at 37°C. The resultant peptide mixtures first go through a cation exchange step for cleaning up the excess reagents, denaturant, and reducing agent, etc. They then go through an avidin column for affinity enrichment of the labeled (cysteine-containing) peptides. Aliquots containing 10 pmol each of the unchanged components are taken from each pool and are dried using a Speedvac. They are reconstituted with mobile phase A prior to LC/MS and MALDI TOP MS analysis.

### Example 16

### LC/MS And LC/MS/MS Peptide Analyses of Inverse ICAT-Labeled Peptide Mixtures

MS analysis of the ICAT labeled peptide mixtures (see Example 15) is carried out as set forth in Example 3 except that a 5-Da window for precursor ion selection is employed.

### Example 17

### MALDI TOF MS Peptide Analysis of Inverse ICAT-Labeled Peptide Mixtures

Aliquots of the Speedvac dried mixture samples from Example 15 are subjected to the same procedure as set forth in Example 4.

### Example 18

### Database Search of Inverse ICAT-Labeled Peptides

Search software PROWL (Proteometrics, New York, NY) and MASCOT (Matrix Science, London, UK) are used to search the protein databases to identify proteins using peptide fingerprints and MS/MS fragments. For searches using peptide fingerprint information, peptide ions exhibiting the inverse labeling pattern of mass shifts between the two inverse labeling experiments are sorted out based on the direction of mass shift (increasing or decreasing). Each list is used separately for a database search to identify the proteins. An iterative search combining the data of ions with inverse labeling pattern from peptide map and MS/MS is also performed. Any ions that demonstrate a clear inverse labeling pattern in the map and are further supported in MS/MS data by their similar fragmentation pattern and fragments with and without mass shifts are identified first using their MS/MS fragments. The peptides associated with the identified proteins are then removed from the list and a second round search is initiated using the masses of the remaining peptides of inverse labeling pattern. For those ions for which no convincing conclusion can be made, a second analysis is performed using the collected sample to obtain MS/MS data. The resulting data are used in the same manner to search the databases for protein identification.

### Example 19

### MS Analysis of Inverse ICAT Labeling Method Using the Six-Protein Model System

The inverse labeling and MS analysis are performed in the same manner as shown in Figure 1 on the six-protein model system where BSA is "down-regulated" by 3-fold and aldolase "up-regulated" by 3-fold. MALDI TOF MS which is performed directly on mixture without any separation, while displaying a large degree of signal overlap, still clearly demonstrates how the inverse labeling strategy helps to quickly identify the peptide signals derived from proteins of differential expression. Without the inverse labeling strategy one would have to evaluate a single spectrum (e.g., Figure 11 (A)) looking for the ± 8/16/24-Da pair for each and every peptide and performing quantitation. Utilizing the inverse labeling strategy one only needs to overlay the two spectra (Figure 11 (A, B)) and perform "zoom and pick" to identify the peaks that show the characteristic mass shift between the two spectra. Very quickly (a few minutes in this case) after this exercise of qualitative comparison, the peaks of the characteristic inverse labeling pattern are identified (e.g., mass labeled peaks). It is apparent that when applying inverse labeling, a quick qualitative comparison of the two data sets can lead to the quick identification of the peptides of interest. Quantitation and PSD or MS/MS analysis for protein identification can then be performed on those peptides. When the same samples are analyzed using an LCQ with on-line RP LC, the characteristic inverse labeling pattern of mass shift is also clearly observed on a number of peptides (Figure 12 (A, B)). The mass shifts vary depending on the number of cysteines in the sequence and the charge state of the peptide being detected. Following data analysis, two lists of peptide masses are quickly generated that are based on the direction of the mass shift. These two lists are used to search the database. Aldolase is exclusively identified using the list of decrease in mass shift, corresponding to an up-regulation of protein expression. BSA is identified using the list of increase in mass shift, corresponding to a down-regulation in protein expression. MS/MS spectra are obtained automatically in data-dependent mode for a number of the peptides. In order to emulate a broad-spectrum situation where multiple proteins may be up- or down-regulated, an iterative search scheme is also applied. In this case we use the combined mass list of all the peptides that show a mass shift, regardless of the direction of the shift. After a protein is identified with high confidence using either the mass list or an MS/MS spectrum (aldolase in our system), all peptides derived from the protein are removed from the mass list. The process is then repeated in order to identify the next protein displaying the mass shift (BSA in this case). It should be pointed out that there are additional information embedded in the MS and MS/MS data. The mass shifts indicate how many cysteins are present in a sequence. When used for database search, this added specificity helps to narrow down the candidate list and increase the efficiency and accuracy of the search results.

It will be understood that various modifications may be made to the embodiments and/or examples disclosed herein. Thus, the above description should not be construed as limiting, but merely as exemplifications of preferred embodiments.

## Claims

1. A method for identifying a differentially expressed protein in two different samples containing a population of proteins comprising:
a) providing two equal protein pools from each of a reference sample and an experimental sample;
b) labeling the protein pools with a substantially chemically identical isotopically different protein labeling reagent for proteins, wherein one pool from each of the reference and experimental pools is labeled with an isotopically heavy protein labeling reagent to provide an isotopically heavy-labeled reference pool and an isotopically heavy-labeled experimental pool, and wherein the remaining reference and experimental pools are labeled with an isotopically light protein labeling reagent to provide an isotopically light-labeled reference pool and an isotopically light-labeled experimental pool;
c) combining the isotopically light-labeled reference pool with the isotopically heavy-labeled experimental pool to provide a first protein mixture;
d) combining the isotopically heavy-labeled reference pool with the isotopically light-labeled experimental pool to provide a second protein mixture;
e) detecting the labeled proteins from each of the two mixtures; and
f) comparing the labeling pattern obtained for the labeled proteins in the first and second mixtures, wherein an inverse labeling pattern of a protein in the second mixture compared with the labeling pattern of the protein in the first mixture is indicative of the differentially expressed protein in the two different samples.

2. The method of claim 1, which further comprises enzymatically or chemically cleaving the labeled proteins in the first and second mixtures to provide peptide mixtures prior to step (e).

3. The method of claim 2, which further comprises sequencing one of the peptides to identify the differentially expressed protein from which the peptide originated.

4. The method of claim 3, wherein sequencing of the peptide is performed utilizing tandem mass spectrometry or post source decay (PSD).

5. The method of claim 1, which further comprises sequencing the differentially expressed protein to identify the protein.

6. The method of claim 5, wherein sequencing of the differentially expressed protein is performed utilizing tandem mass spectrometry or PSD.

7. The method of claim 1, which further comprises separating the labeled proteins from each of the first and second mixtures prior to step (e).

8. The method of claim 7, wherein the step of separating the labeled proteins from the two mixtures is carried out using a technique selected from the group consisting of ammonium sulfate precipitation, isoelectric focusing, size exclusion chromatography, ion exchange chromatography, adsorption chromatography, reverse phase chromatography, affinity chromatography, ultrafiltration, immunoprecipitation and combinations thereof.

9. The method of claim 2, which further comprises separating the labeled peptides from each of the first and second mixtures prior to step (e).

10. The method of claim 9, wherein the step of separating the labeled peptides from the two mixtures is carried out using a technique selected from the group consisting of size exclusion chromatography, ion exchange chromatography, adsorption chromatography, reverse phase chromatography, affinity chromatography, immunoprecipitation and combinations thereof.

11. The method of claim 1, wherein the labeled proteins are detected by mass spectrometry.

12. The method of claim 2, wherein the labeled peptides are detected by mass spectrometry.

13. The method of claim 1, which further comprises subjecting the samples to at least one fractionation technique to reduce the complexity of proteins in the samples prior to step (a).

14. The method of claim 2, which further comprises subjecting the isotopically labeled proteins of the first and second mixtures to at least one fractionation technique to reduce the complexity of proteins in the first and second mixtures prior to cleaving the labeled proteins in the first and second mixtures.

15. The method of claim 13, wherein the fractionation technique is selected from the group consisting of ammonium sulfate precipitation, isoelectric focusing, size exclusion chromatography, ion exchange chromatography, adsorption chromatography, reverse phase chromatography, affinity chromatography, ultrafiltration, immunoprecipitation and combinations thereof.

16. The method of claim 1, wherein the two samples differ in cell type, tissue type, physiological state, disease state, developmental stage, environmental conditions, nutritional conditions, chemical stimuli or physical stimuli.

17. The method of claim 1, wherein the isotopically heavy protein labeling reagent contains a stable heavy isotope selected from the group consisting of ²H, ¹⁴C, ¹⁵N, ¹⁷O, ¹⁸O and ³⁴S.

18. The method of claim 1, wherein the isotopically light protein labeling reagent contains a stable light isotope selected from the group consisting of H, ¹²C, ¹⁴N, ¹⁶O and ³²S.

19. The method of claim 1, wherein the isotopically heavy protein labeling reagent contains ¹⁸O and the isotopically light protein labeling reagent contains ¹⁶O.

20. The method of claim 1, wherein the protein labeling reagent contains an affinity tag.

21. The method of claim 1, wherein the samples are selected from the group consisting of cell homogenates, cell fractions, tissue homogenates, biological fluids, tears, feces, saliva and lavage fluids.

22. The method of claim 1, wherein the differentially expressed protein is selected from the group consisting of cell surface proteins, membrane proteins, cytosolic proteins and organelle proteins.

23. A method for identifying a differentially expressed protein in two different samples containing a population of proteins comprising:
a) providing two equal protein pools from each of a reference sample and an experimental sample;
b) proteolyzing each protein pool during labeling of each of the protein pools with isotopically labeled water, wherein one pool from each of the reference and experimental pools is labeled with ¹⁸O-water to provide an ¹⁸O-labeled reference pool and an ¹⁸O-labeled experimental pool, and wherein the remaining reference and experimental pools are labeled with ¹⁶O-water to provide an ¹⁶O-labeled reference pool and an ¹⁶O-labeled experimental pool;
c) combining the ¹⁶O-labeled reference pool with the ¹⁸O-labeled experimental pool to provide a first mixture containing ¹⁶O- and ¹⁸O-labeled peptides;
d) combining the ¹⁸O labeled reference pool with the ¹⁶O-labeled experimental pool to provide a second mixture containing ¹⁸O- and ¹⁶O-labeled peptides;
e) detecting the labeled peptides from each of the two mixtures; and
f) comparing the labeling pattern obtained for the labeled peptides in the first and second mixtures, wherein an inverse labeling pattern obtained for a peptide in the second mixture compared with the labeling pattern obtained for the peptide in the first mixture is indicative of the differentially expressed protein from which the peptide originated.

24. The method of claim 23, which further comprises separating the labeled peptides in the two mixtures prior to step (e).

25. The method of claim 24, wherein the step of separating the labeled peptides in the two mixtures is carried out using a technique selected from the group consisting of size exclusion chromatography, ion exchange chromatography, adsorption chromatography, reverse phase chromatography, affinity chromatography, immunoprecipitation and combinations thereof.

26. The method of claim 23, wherein detection of the labeled peptides is carried out by mass spectrometry.

27. The method of claim 23, which further comprises sequencing one of the peptides to identify the differentially expressed protein from which the peptide originated.

28. The method of claim 27, wherein sequencing of the peptide is performed utilizing tandem mass spectrometry or PSD.

29. The method of claim 23, which further comprises subjecting the samples to at least one fractionation technique to reduce the complexity of proteins in the samples prior to step (a).

30. The method of claim 23, which further comprises subjecting the labeled peptides of the first and second mixtures to at least one fractionation technique to separate undesirable peptides from the first and second mixtures prior to step (e).

31. The method of claim 29, wherein the fractionation technique is selected from the group consisting of ammonium sulfate precipitation, isoelectric focusing, size exclusion chromatography, ion exchange chromatography, adsorption chromatography, reverse phase chromatography, affinity chromatography, ultrafiltration, immunoprecipitation and combinations thereof.

32. The method of claim 23, wherein the samples are selected from the group consisting of cell homogenates, cell fractions, tissue homogenates, biological fluids, tears, feces, saliva and lavage fluids.

33. The method of claim 23, wherein the differentially expressed protein is selected from the group consisting of cell surface proteins, membrane proteins, cytosolic proteins and organelle proteins.

34. The method of claim 23, wherein the two samples differ in cell type, tissue type, physiological state, disease state, developmental stage, physiological state, environmental conditions, nutritional conditions, chemical stimuli or physical stimuli.

35. A method for identifying a differentially expressed protein in two different samples containing a population of proteins comprising:
a) providing two equal protein pools from each of a reference sample and an experimental sample;
b) proteolyzing the proteins in each of the protein pools to provide peptide pools;
c) labeling each peptide pool with isotopically labeled water, wherein one peptide pool from each of the reference and experimental pools is labeled with ¹⁸O-water to provide an ¹⁸O-labeled reference peptide pool and an ¹⁸O-labeled experimental peptide pool, and wherein the remaining reference and experimental peptide pools are labeled with ¹⁶O-water to provide an ¹⁶O-labeled reference peptide pool and an ¹⁶O-labeled experimental peptide pool;
d) combining the ¹⁶O-labeled reference pool with the ¹⁸O-labeled experimental pool to provide a first mixture containing ¹⁶O- and ¹⁸O-labeled peptides;
e) combining the ¹⁸O-labeled reference pool with the ¹⁶O-labeled experimental pool to provide a second mixture containing ¹⁸O- and ¹⁶O-labeled peptides;
f) detecting the labeled peptides from each of the two mixtures; and
g) comparing the labeling pattern obtained for the labeled peptides in the first and second mixtures, wherein an inverse labeling pattern obtained for a peptide in the second mixture compared with the labeling pattern obtained for the peptide in the first mixture is indicative of the differentially expressed protein from which the peptide originated.

36. The method of claim 35, which further comprises separating the labeled peptides from the first and second mixtures prior to step (f).

37. The method of claim 36, wherein the step of separating the labeled peptides from the two mixtures is carried out using a technique selected from the group consisting of size exclusion chromatography, ion exchange chromatography, adsorption chromatography, reverse phase chromatography, affinity chromatography, immunoprecipitation and combinations thereof.

38. The method of claim 35, wherein detection of the labeled peptides is carried out by mass spectrometry.

39. The method of claim 35, which further comprises sequencing one of the peptides to identify the differentially expressed protein from which the peptide originated.

40. The method of claim 39, wherein sequencing of the peptide is performed utilizing tandem mass spectrometry or PSD.

41. The method of claim 35, which further comprises subjecting the samples to at least one fractionation technique to reduce the complexity of proteins in the samples prior to step (a).

42. The method of claim 35, which further comprises subjecting the labeled peptides of the first and second mixtures to at least one fractionation technique to separate undesirable peptides from the first and second mixtures prior to step (e).

43. The method of claim 41, wherein the fractionation technique is selected from the group consisting of ammonium sulfate precipitation, isoelectric focusing, size exclusion chromatography, ion exchange chromatography, adsorption chromatography, reverse phase liquid chromatography, affinity chromatography, ultrafiltration, immunoprecipitation and combinations thereof.

44. The method of claim 35, wherein the samples are selected from the group consisting of cell homogenates, cell fractions, tissue homogenates, biological fluids, tears, feces, saliva and lavage fluids.

45. The method of claim 35, wherein the differentially expressed protein is selected from the group consisting of cell surface proteins, membrane proteins, cytosolic proteins and organelle proteins.

46. The method of claim 35, wherein the two samples differ in cell type, tissue type, physiological state, disease state, developmental stage, physiological state, environmental conditions, nutritional conditions, chemical stimuli or physical stimuli.

47. A method for identifying a differentially expressed protein in two different samples containing a population of proteins comprising:
a) providing two equal protein pools from each of a reference sample and an experimental sample wherein one pool from each of the reference and experimental pools is produced by cultivation in a medium containing an isotopically heavy-labeled assimilable source to provide an isotopically heavy-labeled reference pool and an isotopically heavy-labeled experimental pool, and wherein the remaining reference and experimental pools are produced by cultivation in a medium containing an isotopically light-labeled assimilable source to provide an isotopically light-labeled reference pool and an isotopically light-labeled experimental pool;
b) combining the isotopically light-labeled reference pool with the isotopically heavy-labeled experimental pool to provide a first protein mixture;
c) combining the isotopically heavy-labeled reference pool with the isotopically light-labeled experimental pool to provide a second protein mixture;
d) detecting the labeled proteins from each of the two mixtures; and
e) comparing the labeling pattern obtained for the labeled proteins in the first and second mixtures, wherein an inverse labeling pattern of a protein in the second mixture compared with the labeling pattern of the protein in the first mixture is indicative of the differentially expressed protein in the two different samples.

48. The method of claim 47, which further comprises enzymatically or chemically cleaving the labeled proteins in the first and second mixtures to provide peptide mixtures prior to step (d).

49. The method of claim 47, wherein the assimilable source is selected from the group consisting of ammonium salts, glucose, water and amino acids.

## Patentansprüche

1. Verfahren zur Identifizierung eines unterschiedlich exprimierten Proteins in zwei unterschiedlichen Proben, die eine Proteinpopulation enthalten, das umfasst:
a) Bereitstellung von zwei gleichen Proteinpools aus jeweils einer Referenzprobe und einer Experimentalprobe,
b) Markierung der Proteinpools mit einem im wesentlichen chemisch identischen isotopisch unterschiedlichen Proteinmarkierungsreagenz für Proteine, worin ein Pool jeweils aus dem Referenz- und Experimentalpool mit einem isotopisch schweren Proteinmarkierungsreagenz unter Bildung eines isotopisch schwer markierten Referenzpools und eines isotopisch schwer markierten Experimentalpools markiert wird und worin die verbleibenden Referenz- und Experimentalpools mit einem isotopisch leichten Proteinmarkierungsreagenz unter Bildung eines isotopisch leicht markierten Referenzpools und eines isotopisch leicht markierten Experimentalpools markiert werden,
c) Kombination des isotopisch leicht markierten Referenzpools mit dem isotopisch schwer markierten Experimentalpool unter Bildung eines ersten Proteingemisches,
d) Kombination des isotopisch schwer markierten Referenzpools mit dem isotopisch leicht markierten Experimentalpool unter Bildung eines zweiten Proteingemisches,
e) Detektion der markierten Proteine jeweils aus den zwei Gemischen, und
f) Vergleich des für die markierten Proteine aus den ersten und zweiten Gemischen erhaltenen Markierungsmusters, worin ein inverses Markierungsmuster eines Proteins im zweiten Gemisch im Vergleich mit dem Markierungsmuster des Proteins im ersten Gemisch das unterschiedlich exprimierte Protein in den zwei unterschiedlichen Proben anzeigt.

2. Verfahren nach Anspruch 1, das ferner die enzymatische oder chemische Spaltung der markierten Proteine in den ersten und zweiten Gemischen unter Bildung von Peptidgemischen vor dem Schritt (e) umfasst.

3. Verfahren nach Anspruch 2, das ferner die Sequenzierung eines der Peptide zur Identifizierung des unterschiedlich exprimierten Proteins umfasst, von dem das Peptid stammt.

4. Verfahren nach Anspruch 3, worin die Sequenzierung des Peptids mittels einer Tandemmassenspektrometrie oder Post-Source-Decay (PSD) ausgeführt wird.

5. Verfahren nach Anspruch 1, das femer die Sequenzierung des unterschiedlich exprimierten Proteins zur Identifizierung des Proteins umfasst.

6. Verfahren nach Anspruch 5, worin die Sequenzierung des unterschiedlich exprimierten Proteins mittels Tandemmassenspektrometrie oder PSD ausgeführt wird.

7. Verfahren nach Anspruch 1, das ferner die Abtrennung der markierten Proteine jeweils aus den ersten und zweiten Gemischen vor Schritt (e) umfasst.

8. Verfahren nach Anspruch 7, worin der Abtrennungsschritt der markierten Proteine aus den zwei Gemischen mittels einer Technik ausgeführt wird, die aus der Gruppe ausgewählt ist, welche besteht aus Ammoniumsulfatfällung, isoelektrischer Fokussierung, Größenausschlusschromatographie, lonenaustauschchromatograpie, Adsorptionschromatographie, Umkehrphasenchromatographie, Affinitätschromatographie, Ultrafiltration, Immunfällung und Kombinationen hiervon.

9. Verfahren nach Anspruch 2, das ferner die Abtrennung der markierten Peptide aus jeweils den ersten und zweiten Gemischen vor dem Schritt (e) umfasst.

10. Verfahren nach Anspruch 9, worin der Abtrennungsschritt der markierten Peptide aus den zwei Gemischen mittels einer Technik ausgeführt wird, die aus der Gruppe ausgewählt ist, welche besteht aus Größenausschlusschromatographie, lonenaustauschchromatograpie, Adsorptionschromatographie, Umkehrphasenchromatographie, Affinitätschromatographie, Immunfällung und Kombinationen hiervon.

11. Verfahren nach Anspruch 1, worin die markierten Proteine durch Massenspektrometrie detektiert werden.

12. Verfahren nach Anspruch 2, worin die markierten Peptide durch Massenspektrometrie detektiert werden.

13. Verfahren nach Anspruch 1, das femer die Anwendung von zumindest einer Fraktionierungstechnik auf die Proben zur Verringerung der Komplexität der Proteine in den Proben vor dem Schritt (a) umfasst.

14. Verfahren nach Anspruch 2, das femer die Anwendung zumindest einer Fraktionierungstechnik auf die isotopisch markierten Proteine der ersten und zweiten Gemische zur Verringerung der Komplexität der Proteine in den ersten und zweiten Gemischen vor der Spaltung der markierten Proteine in den ersten und zweiten Gemischen umfasst.

15. Verfahren nach Anspruch 13, worin die Fraktionierungstechnik aus der Gruppe ausgewählt ist, die besteht aus Ammoniumsulfatfällung, isoelektrischer Fokussierung, Größenausschlusschromatographie, Ionenaustauschchromatograpie, Adsorptionschromatographie, Umkehrphasenchromatographie, Affinitätschromatographie, Ultrafiltration, Immunfällung und Kombinationen hiervon.

16. Verfahren nach Anspruch 1, worin die zwei Proben sich im Zelltyp, Gewebetyp, physiologischen Zustand, Krankheitszustand, Entwicklungszustand, Umgebungsbedingungen, Emährungsbedingungen, chemischen Stimuli oder physikalischen Stimuli unterscheiden.

17. Verfahren nach Anspruch 1, worin das isotopisch schwere Proteinmarkierungsreagenz ein stabiles schweres Isotop ausgewählt aus der Gruppe enthält, die besteht aus ²H, ¹⁴C, ¹⁵N, ¹⁷O, ¹⁸O und ³⁴S.

18. Verfahren nach Anspruch 1, worin das isotopisch leichte Proteinmarkierungsreagenz ein stabiles leichtes Isotop ausgewählt aus der Gruppe enthält, die besteht aus H, ¹²C, ¹⁴N, ¹⁶O und ³²S.

19. Verfahren nach Anspruch 1, worin das isotopisch schwere Proteinmarkierungsreagenz ¹⁸O enthält und das isotopisch leichte Proteinmarkierungsreagenz ¹⁶O enthält.

20. Verfahren nach Anspruch 1, worin das Proteinmarkierungsreagenz einen Affinitätsanhang enthält.

21. Verfahren nach Anspruch 1, worin die Proben aus der Gruppe ausgewählt sind, die besteht aus Zellhomogenaten, Zellfraktionen, Gewebehomogenaten, biologischen Flüssigkeiten, Tränen, Fäkalien, Speichel und Lavageflüssigkeiten.

22. Verfahren nach Anspruch 1, worin das unterschiedlich exprimierte Protein aus der Gruppe ausgewählt ist, die besteht aus Oberflächenproteinen, Membranproteinen, cytosolischen Proteinen und Organellproteinen.

23. Verfahren zur Identifizierung eines unterschiedlich exprimierten Proteins in zwei unterschiedlichen Proben, die eine Proteinpopulation enthalten, das umfasst
a) Bereitstellung von zwei gleichen Proteinpools aus jeweils einer Referenzprobe und einer Experimentalprobe,
b) Proteolyse jedes Proteinpools während der Markierung jedes der Proteinpools mit isotopisch markiertem Wasser, worin jeweils ein Pool der Referenz- und der Experimentalpools mit ¹⁸O-Wasser unter Bildung eines ¹⁸O-markierten Referenzpools und eines ¹⁸O-markierten Experimentalpools markiert wird und worin die verbleibenden Referenz- und Experimentalpools mit ¹⁶O-Wasser unter Bildung eines ¹⁶O-markierten Referenzpools und eines ¹⁶O-markierten Experimentalpools markiert werden,
c) Kombination des ¹⁶O-markierten Referenzpools mit dem ¹⁸O-markierten Experimentalpool unter Bildung eines ersten Gemisches, das ¹⁶O- und ¹⁸O-markierte Peptide enthält,
d) Kombination des ¹⁸O-markierten Referenzpools mit dem ¹⁶O-markierten Experimentalpool unter Bildung eines zweiten Gemisches, das ¹⁸O- und ¹⁶O-markierte Peptide enthält,
e) Detektion der markierten Peptide jeweils aus den zwei Gemischen, und
f) Vergleich der für die markierten Peptide in den ersten und zweiten Gemischen erhaltenen Markierungsmuster, worin ein inverses Markierungsmuster, das für ein Peptid im zweiten Gemisch erhalten wurde, im Vergleich mit dem Markierungsmuster, das für das Peptid im ersten Gemisch erhalten wurde, das unterschiedlich exprimierte Protein anzeigt, von dem das Peptid stammt.

24. Verfahren nach Anspruch 23, das ferner die Abtrennung der markierten Peptide in den zwei Gemischen vor dem Schritt (e) umfasst.

25. Verfahren nach Anspruch 24, worin der Trennschritt der markierten Peptide in den zwei Gemischen mittels einer Technik ausgeführt wird, die aus der Gruppe ausgewählt ist, welche besteht aus Größenausschlusschromatographie, lonenaustauschchromatographie, Adsorptionschromatographie, Umkehrphasenchromatographie, Affinitätschromatographie, Immunfällung und Kombinationen hiervon.

26. Verfahren nach Anspruch 23, worin die Detektion der markierten Peptide durch Massenspektrometrie ausgeführt wird.

27. Verfahren nach Anspruch 23, das ferner die Sequenzierung eines der Peptide zur Identifizierung des unterschiedlich exprimierten Proteins umfasst, von dem das Peptid stammt.

28. Verfahren nach Anspruch 27, worin die Sequenzierung des Peptids mittels Tandemmassenspektrometrie oder PSD ausgeführt wird.

29. Verfahren nach Anspruch 23, das ferner die Anwendung zumindest einer Fraktionierungstechnik auf die Proben zur Verringerung der Komplexität von Proteinen in den Proben vor dem Schritt (a) umfasst.

30. Verwendung nach Anspruch 23, das femer die Anwendung einer Fraktionierungstechnik auf die markierten Peptide der ersten und zweiten Gemische zur Abtrennung von unerwünschten Peptiden aus den ersten und zweiten Gemischen vor dem Schritt (e) umfasst.

31. Verfahren nach Anspruch 29, worin die Fraktionierungstechnik aus der Gruppe ausgewählt wird, die besteht aus Ammoniumsulfatfällung, isoelektrischer Fokussierung, Größenausschlusschromatographie, lonenaustauschchromatograpie, Adsorptionschromatographie, Umkehrphasenchromatographie, Affinitätschromatographie, Ultrafiltration, Immunfällung und Kombinationen hiervon.

32. Verfahren nach Anspruch 23, worin die Proben aus der Gruppe ausgewählt werden, die besteht aus Zellhomogenaten, Zellfraktionen, Gewebehomogenaten, biologischen Flüssigkeiten, Tränen, Fäkalien, Speichel und Lavageflüssigkeiten.

33. Verfahren nach Anspruch 23, worin das unterschiedlich exprimierte Protein aus der Gruppe ausgewählt ist, die besteht aus Zelloberflächenproteinen, Membranproteinen, cytosolischen Proteinen und Organellproteinen.

34. Verfahren nach Anspruch 23, worin die zwei Proben sich im Zelltyp, Gewebetyp, physiologischen Zustand, Krankheitszustand, Entwicklungszustand, Umgebungsbedingungen, Emährungsbedingungen, chemischen Stimuli oder physikalischen Stimuli unterscheiden.

35. Verfahren zur Identifizierung eines unterschiedlich exprimierten Proteins in zwei unterschiedlichen Proben, die eine Proteinpopulation enthalten, das umfasst:
a) Bereitstellung von zwei gleichen Proteinpools aus jeweils einer Referenzprobe und einer Experimentalprobe,
b) Proteolyse der Proteine in jedem Proteinpool unter Bildung von Peptidpools,
c) Markierung jedes Peptidpools mit isotopisch markiertem Wasser, worin jeweils ein Peptidpool der Referenz- und der Experimentalpools mit ¹⁸O-Wasser unter Bildung eines ¹⁸O-markierten Referenzpeptidpools und eines ¹⁸O-markierten Experimentalpeptidpools markiert wird und worin die verbleibenden Referenz- und Experimentalpeptidpools mit ¹⁶O-Wasser unter Bildung eines ¹⁶O-markierten Referenzpeptidpools und eines ¹⁶O-markierten Experimentaipeptidpoots markiert werden,
d) Kombination des ¹⁶O-markierten Referenzpools mit dem ¹⁸O-markierten Experimentalpool unter Bildung eines ersten Gemisches, das ¹⁶O- und ¹⁸O-markierte Peptide enthält,
e) Kombination des ¹⁸O-markierten Referenzpools mit dem ¹⁶O-markierten Experimentalpool unter Bildung eines zweiten Gemisches, das ¹⁸O- und ¹⁶O-markierte Peptide enthält,
f) Detektion der markierten Peptide jeweils aus den zwei Gemischen, und
g) Vergleich der für die markierten Peptide in den ersten und zweiten Gemischen erhaltenen Markierungsmuster, worin ein inverses Markierungsmuster, das für ein Peptid im zweiten Gemisch erhalten wurde, im Vergleich mit dem Markierungsmuster, das für das Peptid im ersten Gemisch erhalten wurde, das unterschiedlich exprimierte Protein anzeigt, von dem das Peptid stammt.

36. Verfahren nach Anspruch 35, das femer die Abtrennung der markierten Peptide aus den ersten und zweiten Gemischen vor dem Schritt (f) umfasst.

37. Verfahren nach Anspruch 36, worin der Abtrennschritt der markierten Peptide aus den zwei Gemischen mittels einer Technik ausgeführt wird, die aus der Gruppe ausgewählt wird, die besteht aus Größenausschlusschromatographie, lonenaustauschchromatograpie, Adsorptionschromatographie, Umkehrphasenchromatographie, Affinitätschromatographie, Immunfällung und Kombinationen hiervon.

38. Verfahren nach Anspruch 35, worin die Detektion der markierten Peptide durch Massenspektrometrie ausgeführt wird.

39. Verfahren nach Anspruch 35, das ferner die Sequenzierung eines der Peptide zur Identifizierung des unterschiedlich exprimierten Proteins umfasst, von dem das Peptid stammt.

40. Verfahren nach Anspruch 39, worin die Sequenzierung des Peptids durch Tandemmassenspektrometrie oder PSD ausgeführt wird.

41. Verfahren nach Anspruch 35, das ferner die Anwendung von zumindest einer Fraktionierungstechnik auf die Proben zur Verringerung der Komplexität der Proteine in den Proben vor dem Schritt (a) umfasst.

42. Verfahren nach Anspruch 35, das ferner die Anwendung zumindest einer Fraktionierungstechnik auf die markierten Peptide der ersten und zweiten Gemische zur Abtrennung von unerwünschten Peptiden aus den ersten und zweiten Gemischen vor Schritt (e) umfasst.

43. Verfahren nach Anspruch 41, worin die Fraktionierungstechnik aus der Gruppe ausgewählt wird, die besteht aus Ammoniumsulfatfällung, isoelektrischer Fokussierung, Größenausschlusschromatographie, lonenaustauschchromatograpie, Adsorptionschromatographie, Umkehrphasenflüssigchromatographie, Affinitätschromatographie, Ultrafiltration, Immunfällung und Kombinationen hiervon.

44. Verfahren nach Anspruch 35, worin die Proben aus der Gruppe ausgewählt werden, die besteht aus Zellhomogenaten, Zellfraktionen, Gewebehomogenaten, biologischen Flüssigkeiten, Tränen, Fäkalien, Speichel und Lavageflüssigkeiten.

45. Verfahren nach Anspruch 35, worin das unterschiedlich exprimierte Protein aus der Gruppe ausgewählt wird, die besteht aus Oberflächenproteinen, Membranproteinen, cytosolischen Proteinen und Organellproteinen.

46. Verfahren nach Anspruch 35, worin die zwei Proben sich im Zelltyp, Gewebetyp, physiologischen Zustand, Krankheitszustand, Entwicklungszustand, Umgebungsbedingungen, Ernährungsbedingungen, chemischen Stimuli oder physikalischen Stimuli unterscheiden.

47. Verfahren zur Identifizierung eines unterschiedlich exprimierten Proteins in zwei unterschiedlichen Proben, die eine Proteinpopulation enthalten, das umfasst:
a) Bereitstellung von zwei gleichen Proteinpools aus jeweils einer Referenzprobe und einer Experimentalprobe, worin ein Pool aus jedem der Referenz- und Experimentalpools durch Kultivierung in einem Medium, das eine isotopisch schwer markierte, assimilierbare Quelle enthält, unter Bildung eines isotopisch schwer markierten Referenzpools und eines isotopisch schwer markierten Experimentalpools hergestellt wird und worin die verbleibenden Referenz- und Experimentalpools durch die Kultivierung in einem Medium, das eine isotopisch leicht markierte, assimilierbare Quelle enthält, unter Bildung eines isotopisch leicht markierten Referenzpools und eines isotopisch leicht markierten Experimentalpools hergestellt werden,
b) Kombination des isotopisch leicht markierten Referenzpools mit dem isotopisch schwer markierten Experimentalpool unter Bildung eines ersten Proteingemisches,
c) Kombination des isotopisch schwer markierten Referenzpools mit dem isotopisch leicht markierten Experimentalpool unter Bildung eines zweiten Proteingemisches,
d) Detektion der markierten Proteine jeweils aus den zwei Gemischen, und
e) Vergleich des für die markierten Proteine aus den ersten und zweiten Gemischen erhaltenen Markierungsmusters, worin ein inverses Markierungsmuster eines Proteins im zweiten Gemisch im Vergleich mit dem Markierungsmuster des Proteins im ersten Gemisch das unterschiedlich exprimierte Protein in den zwei unterschiedlichen Proben anzeigt.

48. Verfahren nach Anspruch 47, das ferner die enzymatische oder chemische Spaltung der markierten Proteine in den ersten und zweiten Gemischen unter Bildung von Peptidgemischen vor dem Schritt (d) umfasst.

49. Verfahren nach Anspruch 47, worin die assimilierbare Quelle aus der Gruppe ausgewählt ist, die besteht aus Ammoniumsalzen, Glucose, Wasser und Aminosäuren.

## Revendications

1. Méthode d'identification d'une protéine exprimée de manière différentielle dans deux échantillons différents contenant une population de protéines consistant à réaliser :
a) deux mélanges identiques de protéines à partir de l'échantillon de référence et de l'échantillon d'expérimentation ;
b) un marquage des mélanges de protéines à l'aide d'un réactif marqueur de protéines qui est substantiellement identique d'un point de vue chimique et différent d'un point de vue isotopique, dans lequel un des mélanges de référence et un des mélanges d'expérimentation sont marqués avec un réactif marqueur de protéine isotopiquement lourd afin de procurer un mélange de référence ayant subi un marquage isotopiquement lourd et un mélange d'expérimentation ayant subi un marquage isotopiquement lourd et dans lequel les mélanges de référence et d'expérimentation restants sont marqués avec un réactif marqueur de protéines isotopiquement léger afin de procurer un mélange de référence ayant subi un marquage isotopiquement léger et un mélange d'expérimentation ayant subi un marquage isotopiquement léger ;
c) une combinaison du mélange de référence ayant subi un marquage isotopiquement léger avec le mélange d'expérimentation ayant subi un marquage isotopiquement lourd afin de procurer un premier mélange de protéines ;
d) une combinaison du mélange de référence ayant subi un marquage isotopiquement lourd avec le mélange d'expérimentation ayant subi un marquage isotopiquement léger afin de procurer un second mélange de protéines ;
e) une détection des protéines marquées dans chacun des deux mélanges ; et
f) une comparaison du tracé du marquage obtenu pour les protéines marquées dans les premier et second mélanges dans laquelle le tracé du marquage inverse d'une protéine dans le second mélange en comparaison avec le tracé du marquage de la protéine dans le premier mélange indique la protéine exprimée de manière différentielle dans les deux échantillons différents.

2. Méthode selon la revendication 1 comprenant également le clivage enzymatique ou chimique des protéines marquées dans les premier et second mélanges afin de procurer des mélanges peptidiques préalablement à l'étape (e).

3. Méthode selon la revendication 2 comprenant également le séquençage d'un des peptides afin d'identifier la protéine exprimée de manière différentielle à partir de laquelle le peptide est originaire.

4. Méthode selon la revendication 3, **caractérisée en ce que** le séquençage du peptide se réalise à l'aide d'une spectrométrie de masse tandem ou d'une décroissance après la source (PSD).

5. Méthode selon la revendication 1 comprenant également le séquençage de la protéine exprimée de manière différentielle afin d'identifier la protéine.

6. Méthode selon la revendication 5, **caractérisée en ce que** le séquençage de la protéine exprimée de manière différentielle se réalise à l'aide d'une spectrométrie de masse tandem ou d'une PSD.

7. Méthode selon la revendication 1 comprenant également la séparation des protéines marquées à partir des premier et second mélange préalablement à l'étape (e).

8. Méthode selon la revendication 7, **caractérisée en ce que** la séparation des protéines marquées à partir des deux mélanges se réalise à l'aide d'une technique sélectionnée à partir du groupe comprenant une précipitation par sulfate d'ammonium, une électrofocalisation, une chromatographie d'exclusion stérique; une chromatographie échangeuse d'ions, une chromatographie d'adsorption, une chromatographie à polarité de phase inversée, une chromatographie par affinité, une ultrafiltration, une immunoprécipitation ainsi qu'une combinaison de ces techniques.

9. Méthode selon la revendication 2 comprenant également la séparation des peptides marqués à partir des premier et second mélange préalablement à l'étape (e).

10. Méthode selon la revendication 9, **caractérisée en ce que** la séparation des peptides marqués à partir des deux mélanges se réalise à l'aide d'une technique sélectionnée à partir du groupe comprenant une chromatographie d'exclusion stérique, une chromatographie échangeuse d'ions, une chromatographie d'adsorption, une chromatographie à polarité de phase inversée, une chromatographie par affinité, une immunoprécipitation ainsi qu'une combinaison de ces techniques.

11. Méthode selon la revendication 1, **caractérisée en ce que** les protéines marquées sont détectées par spectrométrie de masse.

12. Méthode selon la revendication 2, **caractérisée en ce que** les peptides marqués sont détectés par spectrométrie de masse.

13. Méthode selon la revendication 1 comprenant également la soumission des échantillons à au moins une technique de fractionnement afin de réduire la complexité des protéines dans les échantillons préalablement à l'étape (a).

14. Méthode selon la revendication 2 comprenant également la soumission des protéines des premier et second mélanges à au moins une technique de fractionnement afin de réduire la complexité des protéines dans les premier et second mélanges préalablement à l'étape de clivage des protéines dans les premier et second mélanges.

15. Méthode selon la revendication 13, **caractérisée en ce que** la technique de fractionnement est sélectionnée à partir du groupe comprenant une précipitation par sulfate d'ammonium, une électrofocalisation, une chromatographie d'exclusion stérique, une chromatographie échangeuse d'ions, une chromatographie d'adsorption, une chromatographie à polarité de phase inversée, une chromatographie par affinité, une ultrafiltration, une immunoprécipitation ainsi qu'une combinaison de ces techniques.

16. Méthode selon la revendication 1, **caractérisée en ce que** les deux échantillons diffèrent au niveau du type de cellules, du type de tissus, de l'état physiologique, de l'état de la maladie, de l'état d'évolution, des conditions environnementales, des conditions nutritionnelles, des stimulations chimiques ou des stimulations physiques.

17. Méthode selon la revendication 1, **caractérisée en ce que** le réactif marqueur de protéine isotopiquement lourd comprend un isotope lourd et stable sélectionné à partir du groupe composé de ²H, ¹⁴C, ⁵N, ¹⁷O, ¹⁸O et ³⁴S.

18. Méthode selon la revendication 1, **caractérisée en ce que** le réactif marqueur de protéine isotopiquement léger comprend un isotope léger et stable sélectionné à partir du groupe composé de H, ¹²C, ¹⁴N, ¹⁶O et ³²S.

19. Méthode selon la revendication 1, **caractérisée en ce que** le réactif marqueur de protéine isotopiquement lourd comprend ¹⁸O et **en ce que** le réactif marqueur de protéine isotopiquement léger comprend ¹⁶O.

20. Méthode selon la revendication 1, **caractérisée en ce que** le réactif marqueur de protéine contient un marqueur d'affinité.

21. Méthode selon la revendication 1, **caractérisée en ce que** les échantillons sont sélectionnés à partir du groupe comprenant des broyats de cellules, des fractions de cellules, des broyats de tissus, des fluides biologiques, des larmes, des selles, de la salive et des fluides de lavage.

22. Méthode selon la revendication 1, **caractérisée en ce que** la protéine exprimée de manière différentielle est sélectionnée à partir des groupes comprenant des protéines antigéniques, des protéines membranaires, des protéines cytosoliques et des protéines organelles.

23. Méthode d'identification d'une protéine exprimée de manière différentielle dans deux échantillons différents contenant une population de protéines consistant à réaliser :
a) deux mélanges identiques de protéines provenant chacun d'un échantillon de référence et d'un échantillon d'expérimentation ;
b) une protéolyse de chacun des mélanges de protéines au cours de la phase de marquage de chacun des mélanges de protéines avec de l'eau marquée isotopiquement, dans laquelle un des mélanges de référence et d'expérimentation est marqué avec de l'eau ¹⁸O afin de procurer un mélange de référence marqué par ¹⁸O et un mélange d'expérimentation marqué par ¹⁸O et dans laquelle les mélanges de référence et d'expérimentation restants sont marqués avec de l'eau ¹⁶O afin de procurer un mélange de référence marqué par ¹⁶O et un mélange d'expérimentation marqué par ¹⁶O ;
c) une combinaison du mélange de référence marqué par ¹⁶O avec le mélange d'expérimentation marqué par ¹⁸O afin de procurer un premier mélange contenant des peptides marqués par ¹⁶O et ¹⁸O ;
d) une combinaison du mélange de référence marqué par ¹⁸O avec le mélange d'expérimentation marqué par ¹⁶O afin de procurer un second mélange contenant des peptides marqués par ¹⁶O et ¹⁸O ;
e) une détection des peptides marqués dans chacun des deux mélanges ; et
f) une comparaison du tracé du marquage obtenu pour les peptides marqués dans les premier et second mélanges dans laquelle un marquage inverse d'un peptide dans le second mélange en comparaison avec le tracé du marquage obtenu avec le peptide dans le premier mélange indique la protéine exprimée de manière différentielle à partir de laquelle le peptide est originaire.

24. Méthode selon la revendication 23 comprenant également la séparation des peptides marqués dans les deux mélanges préalablement à l'étape (e).

25. Méthode selon la revendication 24, **caractérisée en ce que** l'étape de séparation des peptides marqués dans les deux mélanges est réalisée à l'aide d'une technique sélectionnée à partir du groupe comprenant une chromatographie d'exclusion stérique, une chromatographie échangeuse d'ions, une chromatographie d'adsorption, une chromatographie à polarité de phase inversée, une chromatographie par affinité, une immunoprécipitation ainsi qu'une combinaison de ces techniques.

26. Méthode selon la revendication 23 **caractérisée en ce que** la détection des peptides marqués se réalise par spectrométrie de masse.

27. Méthode selon la revendication 23 comprenant également le séquençage d'un des peptides afin d'identifier la protéine exprimée de manière différentielle à partir de laquelle le peptide est originaire.

28. Méthode selon la revendication 27 **caractérisée en ce que** le séquençage du peptide se réalise à l'aide d'une spectrométrie de masse tandem ou d'une PSD.

29. Méthode selon la revendication 23 comprenant également la soumission des échantillons à au moins une technique de fractionnement afin de réduire la complexité des protéines dans les échantillons préalablement à l'étape (a).

30. Méthode selon la revendication 23 comprenant également la soumission des peptides des premier et second mélanges à au moins une technique de fractionnement afin de séparer les peptides indésirables des premier et second mélanges préalablement à l'étape (e).

31. Méthode selon la revendication 29, **caractérisée en ce que** l'étape de fractionnement est sélectionnée à partir du groupe comprenant une précipitation par sulfate d'ammonium, une électrofocalisation, une chromatographie d'exclusion stérique, une chromatographie échangeuse d'ions, une chromatographie d'adsorption, une chromatographie à polarité de phase inversée, une chromatographie par affinité, une ultrafiltration, une immunoprécipitation ainsi qu'une combinaison de ces techniques.

32. Méthode selon la revendication 23, **caractérisée en ce que** les échantillons sont sélectionnés à partir du groupe comprenant des broyats de cellules, des fractions de cellules, des broyats de tissus, des fluides biologiques, des larmes, des selles, de la salive et des fluides de lavage.

33. Méthode selon la revendication 23, **caractérisée en ce que** la protéine exprimée de manière différentielle est sélectionnée à partir du groupe comprenant des protéines antigéniques, des protéines membranaires, des protéines cytosoliques et des protéines organelles.

34. Méthode selon la revendication 23, **caractérisée en ce que** les deux échantillons différent au niveau du type de cellules, du type de tissus, de l'état physiologique, de l'état de la maladie, de l'état d'évolution, de l'état physiologique, des conditions environnementales, des conditions nutritionnelles, des stimulations chimiques ou des stimulations physiques.

35. Méthode d'identification d'une protéine exprimée de manière différentielle dans deux échantillons différents contenant une population de protéines consistant à réaliser :
a) deux mélanges identiques de protéines provenant chacun d'un échantillon de référence et d'un échantillon d'expérimentation ;
b) une protéolyse des protéines dans chacun des mélanges de protéines afin de procurer des mélanges de peptides ;
c) un marquage de chacun des mélanges de peptides avec de l'eau marquée isotopiquement, dans lequel un des mélanges de peptides de référence et d'expérimentation est marqué avec de l'eau ¹⁸O afin de procurer un mélange de peptides de référence marqué par ¹⁸O et un mélange de peptides d'expérimentation marqué par ¹⁸O et dans lequel les mélanges de peptides de référence et d'expérimentation restants sont marqués avec de l'eau ¹⁶O afin de procurer un mélange de peptides de référence marqués par ¹⁶O et un mélange de peptides d'expérimentation marqués par ¹⁶O ;
d) une combinaison du mélange de référence marqué par ¹⁶O avec le mélange d'expérimentation marqué par ¹⁸O afin de procurer un premier mélange contenant des peptides marqués par ¹⁶O et ¹⁸O ;
e) une combinaison du mélange de référence marqué par ¹⁸O avec le mélange d'expérimentation marqué par ¹⁶O afin de procurer un second mélange contenant des peptides marqués par ¹⁶O et ¹⁸O ;
f) une détection des peptides marqués dans chacun des deux mélanges ; et
g) une comparaison du tracé du marquage obtenu pour les peptides marqués dans les premier et second mélanges dans laquelle un tracé du marquage inverse d'un peptide dans le second mélange en comparaison avec le tracé du marquage d'un peptide dans le premier mélange indique une protéine exprimée de manière différentielle à partir de laquelle le peptide est originaire.

36. Méthode selon la revendication 35 comprenant également la séparation des peptides marqués à partir des premier et second mélanges préalablement à l'étape (f).

37. Méthode selon la revendication 36, **caractérisée en ce que** la séparation des peptides marqués à partir des deux mélanges se réalise à l'aide d'une technique sélectionnée à partir du groupe comprenant une chromatographie d'exclusion stérique, une chromatographie échangeuse d'ions, une chromatographie d'adsorption, une chromatographie à polarité de phase inversée, une chromatographie par affinité, une immunoprécipitation ainsi qu'une combinaison de ces techniques.

38. Méthode selon la revendication 35, **caractérisée en ce que** la détection des peptides marqués se réalise à l'aide d'une spectrométrie de masse.

39. Méthode selon la revendication 35 comprenant également le séquençage d'un des peptides afin d'identifier la protéine exprimée de manière différentielle à partir de laquelle le peptide est originaire.

40. Méthode selon la revendication 39, **caractérisée en ce que** le séquençage du peptide se réalise à l'aide d'une spectrométrie de masse tandem ou d'une PSD.

41. Méthode selon la revendication 35 comprenant également la soumission des échantillons à au moins une technique de fractionnement afin de réduire la complexité des protéines dans les échantillons préalablement à l'étape (a).

42. Méthode selon la revendication 35 comprenant également la soumission des peptides des premier et second mélanges à au moins une technique de fractionnement afin de séparer les peptides indésirables des premier et second mélanges préalablement à l'étape (e).

43. Méthode selon la revendication 41, **caractérisée en ce que** la technique de fractionnement est sélectionnée à partir du groupe comprenant une précipitation par sulfate d'ammonium, une électrofocalisation, une chromatographie d'exclusion stérique, une chromatographie échangeuse d'ions, une chromatographie d'adsorption, une chromatographie à polarité de phase inversée, une chromatographie par affinité, une ultrafiltration, une immunoprécipitation ainsi qu'une combinaison de ces techniques.

44. Méthode selon la revendication 35, **caractérisée en ce que** les échantillons sont sélectionnés à partir du groupe comprenant des broyats de cellules, des fractions de cellules, des broyats de tissus, des fluides biologiques, des larmes, des selles, de la salive et des fluides de lavage.

45. Méthode selon la revendication 35, **caractérisée en ce que** la protéine exprimée de manière différentielle est sélectionnée à partir des groupes comprenant des protéines antigéniques, des protéines membranaires, des protéines cytosoliques et des protéines organelles.

46. Méthode selon la revendication 35, **caractérisée en ce que** les deux échantillons différent au niveau du type de cellules, du type de tissus, de l'état physiologique, de l'état de la maladie, de l'état d'évolution, des conditions environnementales, des conditions nutritionnelles, des stimulations chimiques ou des stimulations physiques.

47. Méthode d'identification d'une protéine exprimée de manière différentielle dans deux échantillons différents contenant une population de protéines consistant à réaliser :
a) deux mélanges identiques de protéines provenant chacun d'un échantillon de référence et d'un échantillon d'expérimentation dans lesquels un des mélanges de référence et d'expérimentation est produit par mise en culture dans un milieu contenant une source assimilable ayant subi un marquage isotopiquement lourd afin de procurer un mélange de référence ayant subi un marquage isotopiquement lourd et un mélange d'expérimentation ayant subi un marquage isotopiquement lourd et dans lesquels les mélanges de référence et d'expérimentation restants sont produits par mise en culture dans un milieu contenant une source assimilable ayant subi un marquage isotopiquement léger afin de procurer un mélange de référence ayant subi un marquage isotopiquement léger et un mélange d'expérimentation ayant subi un marquage isotopiquement léger ;
b) une combinaison du mélange de référence ayant subi un marquage isotopiquement léger avec le mélange d'expérimentation ayant subi un marquage isotopiquement lourd afin de procurer un premier mélange de protéines ;
c) une combinaison du mélange de référence ayant subi un marquage isotopiquement lourd avec le mélange d'expérimentation ayant subi un marquage isotopiquement léger afin de procurer un second mélange de protéines ;
d) une détection des protéines marquées dans chacun des deux mélanges ; et
e) une comparaison du tracé du marquage obtenu pour les protéines marquées dans les premier et second mélanges dans laquelle un tracé du marquage inverse d'une protéine dans le second mélange en comparaison avec un tracé du marquage de la protéine dans le premier mélange indique la protéine exprimée de manière différentielle dans les deux échantillons différents.

48. Méthode selon la revendication 47 comprenant également un clivage enzymatique ou chimique des protéines marquées dans les premiers et second mélanges afin de procurer des mélanges peptidiques préalablement à l'étape (d).

49. Méthode selon la revendication 47, **caractérisée en ce que** la source assimilable est sélectionnée à partir du groupe comprenant des sels d'ammonium, du glucose, des l'eau et des acides aminés.
